# EUROPEAN PATENT APPLICATION

(11) **EP 4 772 158 A1**
(43) Date of publication of application: **08.07.2026**
(21) Application number: 25223457.0
(22) Date of filing: 15.12.2025
(51) Int. Cl.: A61J 1/20, A61M 5/178

(54) **HAZARDOUS DRUG LIQUID-GAS EXCHANGE DEVICE**

(30) Priority: 02.01.2025 US 202563741177 P
(71) Applicant: Tsai, Hsi-Chin, 238 New Taipei City (TW)
(72) Inventor: Tsai, Hsi-Chin, 238 Taipei City (TW); Chen, Wen-Chieh, 238 New Taipei City (TW)
(74) Representative: Stevens Hewlett & Perkins

(57) **Abstract**

A hazardous drug liquid-gas exchange device includes a syringe, a first connector, a second connector and a pressure balancing device. During the procedure of adding medicine, the collecting portion collects toxic gases so that the atmospheric pressure inside the drug bottle or soft bag maintains pressure balance with the external atmospheric pressure, and also prevents a small amount of harmful drugs inside the drug bottle or soft bag from passing through the second connector in gas form or by evaporation. During the procedure of taking medicine, the collecting portion collects and provides clean air, so that the air pressure inside the drug bottle or soft bag maintains a pressure balance with the external air pressure, and hazardous medicines can be easily taken out from the inside of the drug bottle or soft bag. Hazardous drugs will be blocked by the hydrophobic breathable membrane.

## Description

### CROSS-REFERENCE TO RELATED APPLICATION

This application claims the priority of Taiwanese patent application No. 63/741,177, filed on January 2, 2025, which is incorporated herewith by reference.

### BACKGROUND OF THE INVENTION

### 1. Field of the Invention

The present invention relates generally to a drug-dispensing and infusion system, and more particularly to a hazardous drug liquid-gas exchange device for dispensing hazardous drugs contained in a drug bottle or soft bag and for preventing the leakage of toxic gases.

### 2. The Prior Arts

Some drugs are toxic and can harm the health of healthcare workers; these are called hazardous drugs, such as chemotherapy drugs used to treat cancer. To reduce the risk of healthcare workers being exposed to hazardous drugs, the sealed transfer of these drugs is crucial. The outer surface of the needle-free connector must be completely free of any hazardous drug residue, and toxic gases containing hazardous drugs must not be allowed to disperse into the external space. Otherwise, accidental exposure of health workers to hazardous drugs can affect the nervous system, damage the reproductive system, and increase the risk of leukemia.

The following sections will describe three conventional drug-dispensing and infusion systems.

The first conventional drug-dispensing and infusion system includes a syringe, a base, and a needle-free connector. The syringe includes a syringe barrel, a plunger, and a piston. The plunger is disposed within the syringe barrel and has a plunger handle at its tip. The piston is disposed at the bottom end of the plunger and is located within the syringe barrel. The base includes a puncture portion with a pointed tip. The needle-free connector is disposed between the syringe barrel and the base.

The drug-adding procedure of the first conventional drug-dispensing and infusion system includes the following steps: the tip of the puncture portion punctures a stopper of a drug bottle or a membrane at the infusion end of a soft bag; the first conventional drug-dispensing and infusion system is on top, with the drug bottle or soft bag below; the plunger handle is pushed, the plunger drives the piston to move inward, and the piston injects the hazardous drug inside the syringe from top to bottom sequentially through the needle-free connector and the base into the drug bottle or soft bag. However, because the drug bottle is usually made of a hard and non-deformable material such as glass, the atmospheric pressure inside the drug bottle will be greater than the atmospheric pressure outside when the hazardous drug is injected into the drug bottle. If the syringe is separated from the needle-free connector at this time, the gas inside the drug bottle will be depressurized through the needle-free connector, causing a small amount of hazardous drug inside the drug bottle to pass through the needle-free connector in gas form or by evaporation, which easily makes the hazardous drug remaining on the outer surface of the needle-free connector, and the toxic gas containing the hazardous drug will also drift into the external space through the needle-free connector. At this time, healthcare workers may come into contact with the hazardous drug through their skin, and may also inhale toxic gases into their lungs, affecting the nervous system, damaging the reproductive system, and increasing the risk of leukemia.

The drug-dispensing procedure of the first conventional drug-dispensing and infusion system includes the following steps: pushing the plunger handle, the plunger drives a piston to move inward, the piston expelling air from inside the syringe sequentially through the needle-free connector and the base; the pointed tip of the puncture portion punctures the stopper of the drug bottle or the thin film of the infusion end of the soft bag; with the drug bottle or soft bag on top and the first conventional drug-dispensing and infusion system below, pulling the plunger handle, the plunger drives the piston to move outward, causing the hazardous drug inside the drug bottle or soft bag to be injected into the syringe from top to bottom through the base and the needle-free connector. However, during the drug-dispensing procedure, because the first conventional drug-dispensing and infusion system does not inject clean air into the drug bottle or soft bag, the drug bottle or soft bag cannot achieve pressure balance, making it difficult to remove the hazardous drug from inside the drug bottle or soft bag.

The difference between the second conventional drug-dispensing and infusion system and the first conventional drug-dispensing and infusion system is that the second conventional drug-dispensing and infusion system further includes a balloon, which is disposed on one side of the base.

The drug-adding procedure of the second conventional drug-dispensing and infusion system includes the following steps: the pointed tip of the puncture portion punctures the stopper of the drug bottle or the thin film of the infusion end of the soft bag; with the second conventional drug-dispensing and infusion system on top and the drug bottle or soft bag below, the plunger handle is pushed, the plunger drives the piston to move inward, and the piston injects the hazardous drug inside the syringe sequentially from top to bottom through the needle-free connector and the base into the drug bottle or soft bag, while simultaneously the toxic gas inside the drug bottle or soft bag is sequentially injected into the balloon through the needle-free connector and the base, and the balloon continues to inflate. As such, during drug-adding, the second conventional drug-dispensing and infusion system can collect toxic gases via a balloon, maintaining pressure balance between the internal and external atmospheric pressures of the drug bottle or soft bag. This also prevents small amounts of hazardous drug inside the drug bottle or soft bag from passing through the needle-free connector in gaseous or evaporated form. No hazardous drug residue remains on the outer surface of the needle-free connector, and toxic gases do not diffuse into the external space through the connector. Healthcare workers will not come into contact with hazardous drugs on their skin, nor will they inhale toxic gases into their lungs.

The drug-dispensing procedure of the second conventional drug-dispensing and infusion system includes the following steps: Pushing the plunger handle, the plunger drives the piston inward, and the piston injects clean air from inside the syringe sequentially through the needle-free connector and the base into the balloon, so that the balloon contains clean air; the pointed tip of the puncture portion punctures the stopper of the drug bottle or the thin film of the infusion end of the soft bag; with the drug bottle or soft bag on top and the second conventional drug-dispensing and infusion system below, pulling the plunger handle, the plunger drives the piston outward, so that the hazardous drug inside the drug bottle or soft bag is injected from top to bottom sequentially through the base and the needle-free connector into the syringe, while simultaneously, clean air from inside the balloon is injected sequentially through the base and the needle-free connector into the drug bottle or soft bag. As such, during the drug-dispensing procedure, the second conventional drug-dispensing and infusion system can collect and supply clean air through the balloon, maintaining pressure balance between the atmospheric pressure inside the drug bottle or soft bag and the external atmospheric pressure, and making it easy to remove the hazardous drug from inside the drug bottle or soft bag.

With the correct drug-adding and dispensing procedures described above, hazardous drugs will not enter the balloon through the base. However, some uninformed healthcare personnel may place the second conventional drug infusion system at the bottom and the drug bottle or soft bag at the top during drug-adding, causing the hazardous drugs inside the drug bottle or soft bag to easily flow from top to bottom through the base into the balloon.

The structural difference between the third conventional drug-dispensing and infusion system and the second conventional drug-dispensing and infusion system is that the third conventional drug-dispensing and infusion system further includes a check valve, which is located between the base and the balloon.

The difference in the drug-adding procedure between the third conventional drug-dispensing and infusion system and the second conventional drug-dispensing and infusion system is that the toxic gas inside the drug bottle or soft bag passes sequentially through the needle-free connector and the base, smoothly through the check valve, and then is injected into the balloon, which continues to inflate.

The difference between the drug-dispensing procedure of the third conventional drug-dispensing and infusion system and the drug-dispensing procedure of second conventional drug-dispensing and infusion system lies in: pushing the plunger handle causes the plunger to move the piston inward, which expels clean air from inside the syringe. The clean air is then injected from the external space, sequentially from top to bottom, through the base and the needle-free connector into the inside of the drug bottle or soft bag.

However, some uninformed healthcare workers may place the third conventional drug-dispensing and infusion system at the bottom and the drug bottle or soft bag at the top during drug-adding. This causes hazardous drugs inside the drug bottle or soft bag to easily flow from top to bottom through the base and smoothly through the check valve, before being injected into the balloon.

Furthermore, check valves have many shortcomings, including complex structure, large size, high cost, and susceptibility to damage.

### SUMMARY OF THE INVENTION

A primary objective of the present invention is to provide a hazardous drug liquid-gas exchange device. During the drug-adding procedure, a collecting portion can collect toxic gases, maintaining a pressure balance between the internal atmospheric pressure of the drug bottle or soft bag and the external atmospheric pressure. This also prevents small amounts of hazardous drugs inside the drug bottle or soft bag from passing through the needle-free connector in gaseous form or by evaporation.

Another objective of the present invention is to provide a hazardous drug liquid-gas exchange device. During the drug-dispensing procedure, the collecting portion can collect and provide clean air, maintaining a pressure balance between the internal atmospheric pressure of the drug bottle or soft bag and the external atmospheric pressure. Furthermore, the hazardous drugs can be easily removed from the drug bottle or soft bag.

Yet another objective of the present invention is to provide a hazardous drug liquid-gas exchange device in which hazardous drugs cannot enter the collecting portion.

A further objective of the present invention is to provide a hazardous drug liquid-gas exchange device in which the hydrophobic and breathable membrane has advantages such as simple structure, small size, low cost, and resistance to damage.

To achieve the aforementioned objectives, the present invention provides a hazardous drug liquid-gas exchange device, comprising a syringe, a first connector, a second connector, and a pressure balancing device. The syringe includes a syringe barrel, a plunger, and a piston. The plunger is disposed within the syringe barrel and has a plunger handle at its top end. The piston is disposed at the bottom end of the plunger and located within the syringe barrel. The first connector is disposed at the bottom end of the syringe barrel. The second connector is connected to or separate from the first connector and has a puncture portion, and includes a first flow channel and a second flow channel. The bottom end of the puncture portion has a pointed tip, and the first and second flow channels penetrate the bottom end of the puncture portion. The pressure balancing device includes a guide portion, a collecting portion, and a hydrophobic and breathable membrane. The guide portion is disposed on the first connector and/or the second connector and communicates with the first flow channel. The collecting portion communicates with the guide portion. The hydrophobic and breathable membrane is disposed on the guide portion and/or the collecting portion.

The advantages of the present invention are that, during drug-adding procedure, the present invention can collect toxic gases through the collecting portion, maintaining a pressure balance between the internal atmospheric pressure of the drug bottle or soft bag and the external atmospheric pressure. This also prevents small amounts of hazardous drug inside the drug bottle or soft bag from passing through the second connector in gaseous form or by evaporation. No hazardous drug remains on the outer surface of the second connector, and toxic gases will not disperse into the external space through the second connector. Medical personnel will not come into contact with hazardous drugs on their skin, nor will they inhale toxic gases into their lungs.

Furthermore, during the drug-dispensing procedure, the present invention can collect and provide clean air through the collecting portion, maintaining a pressure balance between the internal atmospheric pressure of the drug bottle or soft bag and the external atmospheric pressure. Hazardous drugs are easily removed from the drug bottle or soft bag.

Moreover, some uninformed medical personnel may place the present invention at the bottom and the drug bottle or soft bag at the top during the drug-adding procedure, causing hazardous drugs inside the drug bottle or soft bag to easily pass through the first flow channel and the guide unit sequentially from top to bottom. At this time, the hazardous chemicals will be blocked by the hydrophobic and breathable membrane, preventing the hazardous drugs from passing through and thus preventing them from entering the collection section.

Finally, the hydrophobic and breathable membrane has advantages such as simple structure, small size, low cost, and resistance to damage.

### BRIEF DESCRIPTION OF THE DRAWINGS

The present invention will be apparent to those skilled in the art by reading the following detailed description of a preferred embodiment thereof, with reference to the attached drawings, in which:
FIG. 1 is a perspective view of the first embodiment of the present invention.
FIG. 2 is an exploded view of the first embodiment of the present invention.
FIG. 3A is a cross-sectional view along line III-III of FFIG. 1.
FIG. 3B is a partially enlarged view of FIG. 3A.
FIG. 4 is a cross-sectional view of the first connector and the needle-free connector separated in the first embodiment of the present invention.
FIG. 5 is a cross-sectional view along line V-V of FIG. 1, wherein the fastener secures the drug bottle.
FIG. 6 is a schematic view of drug-adding in the first embodiment of the present invention.
FIG. 7 is a schematic view of drug-dispensing in the first embodiment of the present invention.
FIG. 8 is a perspective view of the second embodiment of the present invention.
FIG. 9 is an exploded view of the second embodiment of the present invention.
FIG. 10A is a cross-sectional view along line X-X of FIG. 8.
FIG. 10B is a partially enlarged view of FIG. 10A.
FIG. 11A is a schematic diagram of drug-adding in the second embodiment of the present invention.
FIG. 11B is a schematic view of region A in FIG. 11A.
FIG. 11C is a schematic view of region B in FIG. 11A.
FIG. 11D is a schematic view of region C in FIG. 11A.
FIG. 12A is a schematic view of drug-dispensing according to the second embodiment of the present invention.
FIG. 12B is a schematic view of region D in FIG. 12A.
FIG. 12C is a schematic view of region E in FIG. 12A.
FIG. 12D is a schematic view of region F in FIG. 12A.
FIG. 13 is a perspective view of the third embodiment of the present invention.
FIG. 14 is an exploded view of the third embodiment of the present invention.
FIG. 15A is a cross-sectional view along line XV-XV in FIG. 13.
FIG. 15B is a partially enlarged view of FIG. 15A.
FIG. 16 is a schematic view of drug-adding according to the third embodiment of the present invention.
FIG. 17 is a schematic view of drug-dispensing according to the third embodiment of the present invention, wherein clean air from the first chamber is injected into the drug bottle.
FIG. 18 is a schematic view of drug-dispensing according to the third embodiment of the present invention, wherein clean air from the external space is injected into the drug bottle using a check valve.
FIG. 19 is a cross-sectional view of the fourth embodiment of the present invention.
FIG. 20 is a cross-sectional view of the fifth embodiment of the present invention.
FIG. 21 is a perspective view of the sixth embodiment of the present invention.
FIG. 22 is an exploded view of the sixth embodiment of the present invention.
FIG. 23 is a cross-sectional view along line XXIII-XXIII of FIG. 21.
FIG. 24 is a cross-sectional view along line XXIV-XXIV of FIG. 21.
FIG. 25 is a cross-sectional view of the fastener securing the drug bottle according to the sixth embodiment of the present invention.
FIG. 26 is a schematic view of drug-adding according to the sixth embodiment of the present invention.
FIG. 27 is a schematic dview of drug-dispensing according to the sixth embodiment of the present invention.
FIG. 28 is a perspective view of the seventh embodiment of the present invention.
FIG. 29 is an exploded view of the seventh embodiment of the present invention.
FIG. 30 is a cross-sectional view along line XXX-XXX of FIG. 28.
FIG. 31 is a schematic view of drug-dispensing according to the seventh embodiment of the present invention, wherein clean air from the external space is injected into the drug bottle using a check valve.
FIG. 32 is a perspective view of the eighth embodiment of the present invention.
FIG. 33 is an exploded view of the eighth embodiment of the present invention.
FIG. 34 is a cross-sectional view along line XXXIV-XXXIV of FIG. 32.
FIG. 35 is a perspective view of the ninth embodiment of the present invention.
FIG. 36 is an exploded view of the ninth embodiment of the present invention.
FIG. 37 is a cross-sectional view along line XXXVII-XXXVII of FIG. 35.
FIG. 38A is a partially enlarged view of FIG. 37.
FIG. 38B is a schematic view of region G in FIG. 38A.
FIG. 39A is a cross-sectional view along line XXXIX-XXXIX of FIG. 35.
FIG. 39B is a schematic view of region H in FIG. 39A.
FIG. 40 is a schematic view of the first end of the present invention rotating arbitrarily relative to the second end along the first and second directions.

### DETAILED DESCRIPTION OF THE PREFERRED EMBODIMENT

The accompanying drawings are included to provide a further understanding of the invention, and are incorporated in and constitute a part of this specification. The drawings illustrate embodiments of the invention and, together with the description, serve to explain the principles of the invention.

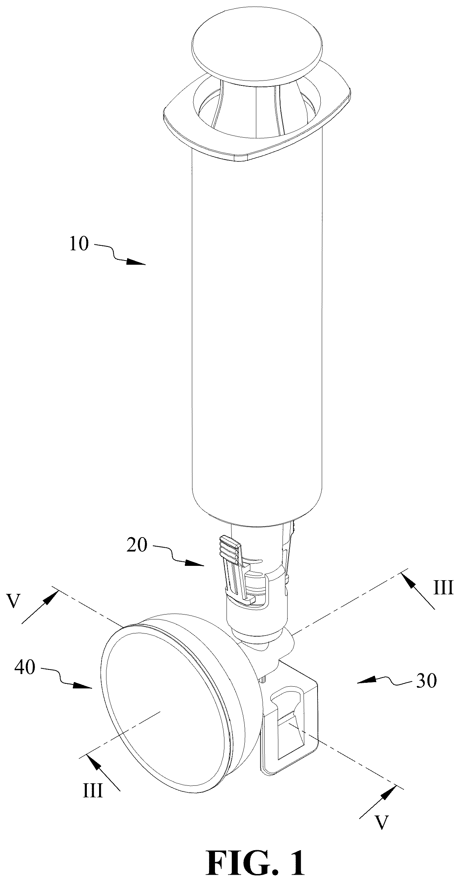

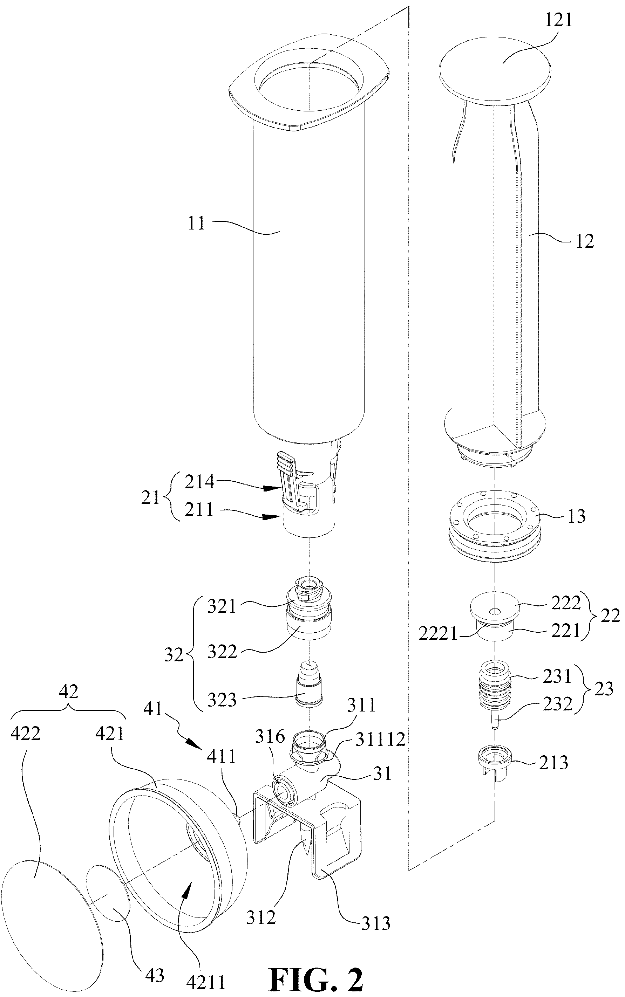

As shown in FIGS. 1, 2, 3A, 3B, and 4, the present invention provides a hazardous drug liquid-gas exchange device, comprising a syringe 10, a first connector 20, a second connector 30, and a pressure balancing device 40.

The syringe 10 includes a syringe barrel 11, a plunger 12, and a piston 13. The plunger 12 is disposed within the syringe barrel 11 and has a plunger handle 121 at its top end. The piston 13 is disposed at the bottom end of the plunger 12 and is located within the syringe barrel 11.

The first connector 20 is a movable closed connector, comprising a body 21, a first engaging portion 22, and a first elastic valve 23. The body 21 includes a sleeve 211, a conduit 212, a positioning portion 213, and two first snap-fit portions 214. The sleeve 211 includes an upper sleeve portion 2111, a middle sleeve portion 2112, and a lower sleeve portion 2113. The upper sleeve portion 2111 is integrally formed with the bottom end of the syringe 11. The inner side of the upper sleeve portion 2111 has a ring groove 21113. The middle sleeve portion 2112 is disposed between the upper sleeve portion 2111 and the lower sleeve portion 2113. A first through hole 2114 is formed between the upper sleeve portion 2111 and the middle sleeve portion 2112, and a second through hole 2115 is formed between the middle sleeve portion 2112 and the lower sleeve portion 2113. The conduit 212 extends from the inside of the upper sleeve portion 2111 to the inside of the middle sleeve portion 2112 and has a first channel 2121 and a first opening 2122. The positioning portion 213 is located inside the upper sleeve portion 2111 and disposed outside the conduit 212. Each first snap-fit portion 214 includes an arm portion 2141, a connecting portion 2142, and a hook portion 2143. The connecting portion 2142 is disposed on the upper sleeve portion 2111, passes through the first through hole 2114 from the inside of the upper sleeve portion 2111, and connects to the arm portion 2141. The hook portion 2143 is disposed at one end of the arm portion 2141 and is located in the second through hole 2115. The first engaging portion 22 includes a first end 221 and a second end 222 and has a connecting channel 223. The first end 221 is located at the top end of the upper sleeve portion 2111 and has a groove 2211. The groove wall of the groove 2211 is arc-shaped. The second end 222 is a female Luer connector and has a protruding ring 2221. The protruding ring 2221 is embedded in the ring groove 21113, so that the second end 222 is close to the bottom of the syringe 11. The connecting channel 223 passes through the first end 221 and the second end 222 and communicates with the groove 2211. The first elastic valve 23 includes a first circumferential portion 231 and a first sealing portion 232. The two ends of the first circumferential portion 231 are respectively disposed on the positioning portion 213 and the first end 221. The first sealing portion 232 is located in the first channel 2121. The top end of the first sealing portion 232 is connected to the inner side of the first circumferential portion 231 and has a plurality of perforations 233. These perforations 233 communicate with the first channel 2121 and the groove 2211. The bottom end of the first sealing portion 232 closes the first opening 2122.

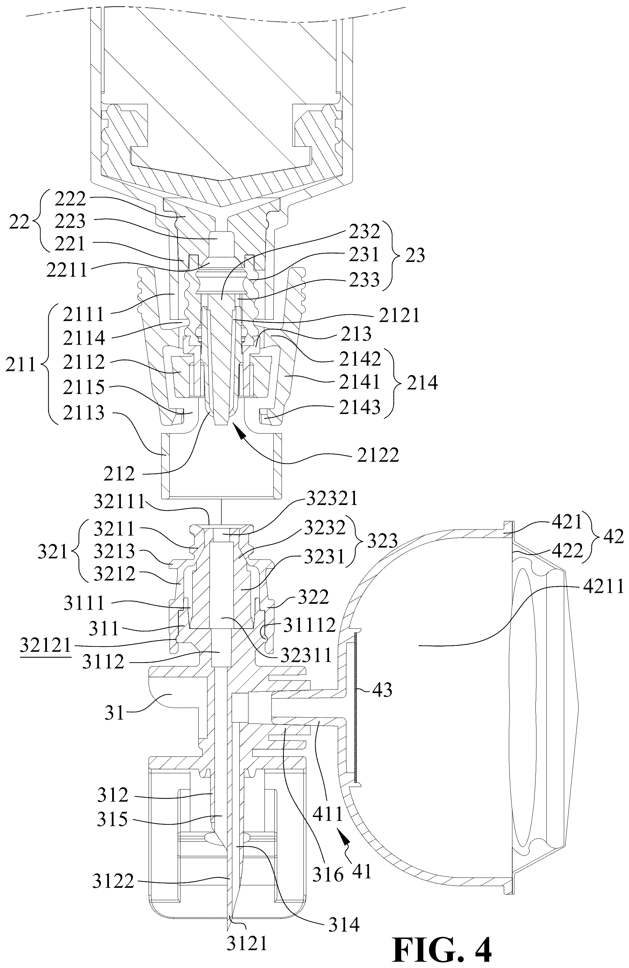

The second connector 30 is a snap-fit, closed, needle-free connector module. The second connector 30 includes a base 31 and a needle-free connector 32. The base 31 includes an assembly portion 311, a piercing portion 312, a fastener 313, and a first protruding portion 316, and has a first flow channel 314 and a second flow channel 315. The assembly portion 311 has an assembly groove 3111 and a shaft hole 3112. The outer side of the assembly portion 311 has a protruding ring 31112. The bottom end of the piercing portion 312 has a pointed tip 3121. The first flow channel 314 passes through the bottom end of the piercing portion 312. The second flow channel 315 communicates with the shaft hole 3112 and passes through the bottom end of the piercing portion 312. A partition wall 3122 is provided between the first flow channel 314 and the second flow channel 315. The pointed tip 3121 is located at the center of the bottom end of the partition wall 3122. The fastener 313 is disposed on the outside of the piercing portion 312. The needle-free connector 32 includes a housing 321, a second engaging portion 322, and a second elastic valve 323. The housing 321 includes an upper housing 3211, a lower housing 3212, and a second snap-fit portion 3213. The top end of the upper housing 3211 has a second opening 32111. The outer diameter of the upper housing 3211 is smaller than the outer diameter of the lower housing 3212. The inner side of the lower housing 3212 has a ring groove 32121, and a protruding ring 31112 is embedded in the ring groove 32121. The second snap-fit portion 3213 is an annular protrusion and is located on the outer side of the top end of the lower housing 3212. The second engaging portion 322 is located on the outer side of the bottom end of the lower housing 3212. The second engaging portion 322 is fastened to the outer side of the assembly portion 311 by a snap-fit manner, and the inner side of the second engaging portion 322 and the outer side of the assembly portion 311 are fully fitted around the circumference to achieve a sealing effect (see FIG. 4). The second elastic valve 323 includes a second circumferential portion 3231 and a second sealing portion 3232. The second circumferential portion 3231 is disposed in the assembly groove 3111 and has a second channel 32311, which communicates with the shaft hole 3112. The second sealing portion 3232 is disposed inside the upper housing 3211 and has a slit 32321, which closes the second opening 32111.

The pressure balancing device 40 includes a guide portion 41, a collecting portion 42, and a hydrophobic and breathable membrane 43. The guide portion 41 includes a tube body 411, which is connected to the first protruding portion 316. The inside of the tube body 411 communicates with the first flow channel 314. The collecting portion 42 includes an outer cover 421 and an airbag 422. The outer cover 421 is integrally formed with one end of the tube body 411. The outer cover 421 has a first chamber 4211. The inside of the tube body 411 communicates with the first chamber 4211. The hydrophobic and breathable membrane 43 is disposed in the first chamber 4211 and near the opening of the tube body 411. The airbag 422 is disposed at the opening of the first chamber 4211.

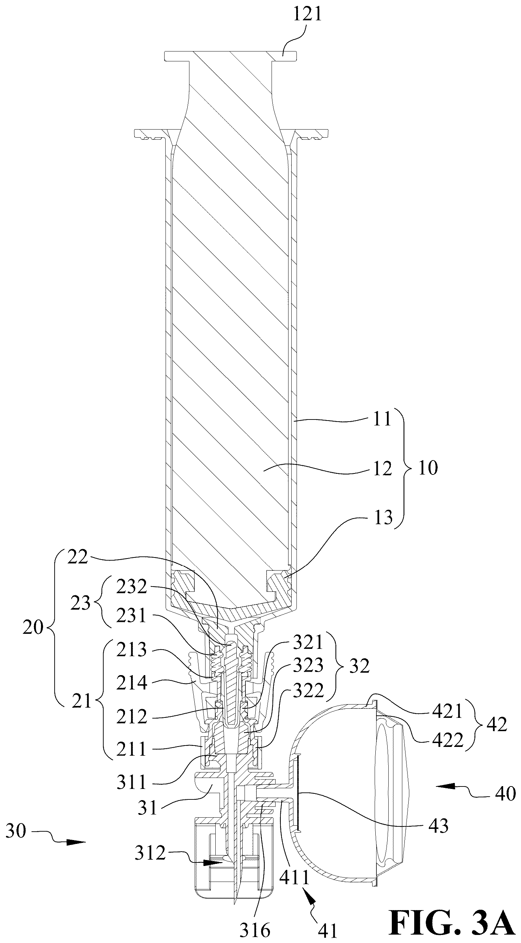

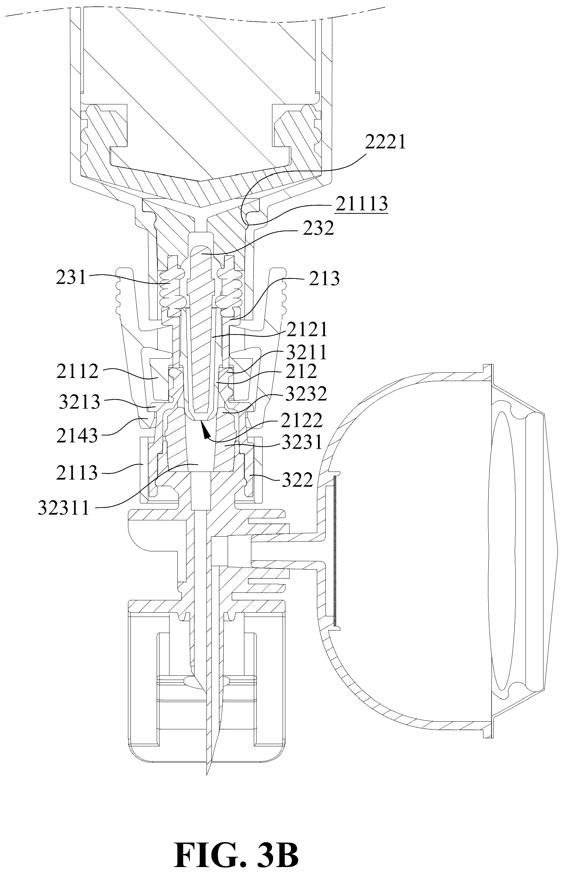

As shown in FIGS. 3A and 3B, when the first connector 20 mates with the needle-free connector 32, the second snap-fit portion 3213 is located in the second through hole 2115 (see FIG. 4), the hook portion 2143 is fixed to the bottom end of the second snap-fit portion 3213, the conduit 212 compresses the second sealing portion 3232, and the second sealing portion 3232 compresses the second circumferential portion 3231, so that the second sealing portion 3232 moves away from the second opening 32111 (see FIG. 4), and the conduit 212 passes through the slit 32321 (see FIG. 4) and enters the inside of the second sealing portion 3232. The upper housing 3211 abuts against the inner wall of the middle sleeve portion 2112. The positioning portion 213 moves along the conduit 212 away from the needle-free connector 32 and compresses the first circumferential portion 231, so that the bottom end of the first sealing portion 232 disengages from the first opening 2122, and the first channel 2121 communicates with the second channel 32311. The inner side of the lower sleeve portion 2113 abuts against the outer side of the second engaging portion 322, thus preventing the lower housing 3212 from wobbling within the lower sleeve portion 2113.

As shown in FIG. 4, when the first connector 20 separates from the needle-free connector 32, pressing the arm portion 2141 causes the hook portion 2143 to disengage from the bottom end of the second snap-fit portion 3213. The conduit 212 disengages from the second sealing portion 3232, allowing the slit 32321 to seal. The second sealing portion 3232 is reset by the elasticity of the second circumferential portion 3231 and closes the second opening 32111. The upper housing 3211 disengages from the middle sleeve portion 2112. The positioning portion 213 and the first sealing portion 232 are reset by the elasticity of the first circumferential portion 231, causing the bottom end of the first sealing portion 232 to close the first opening 2122.

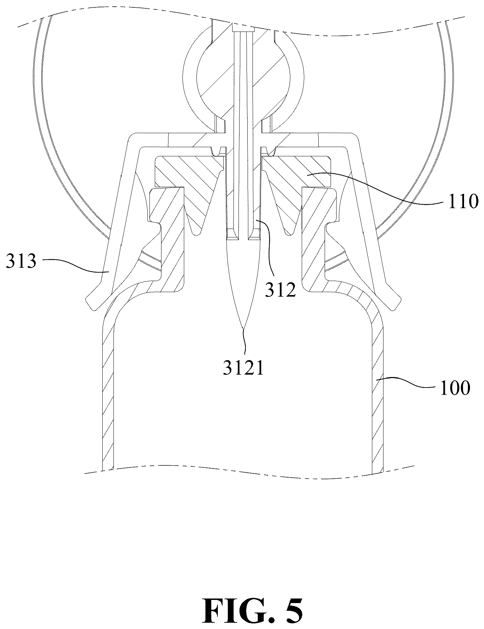

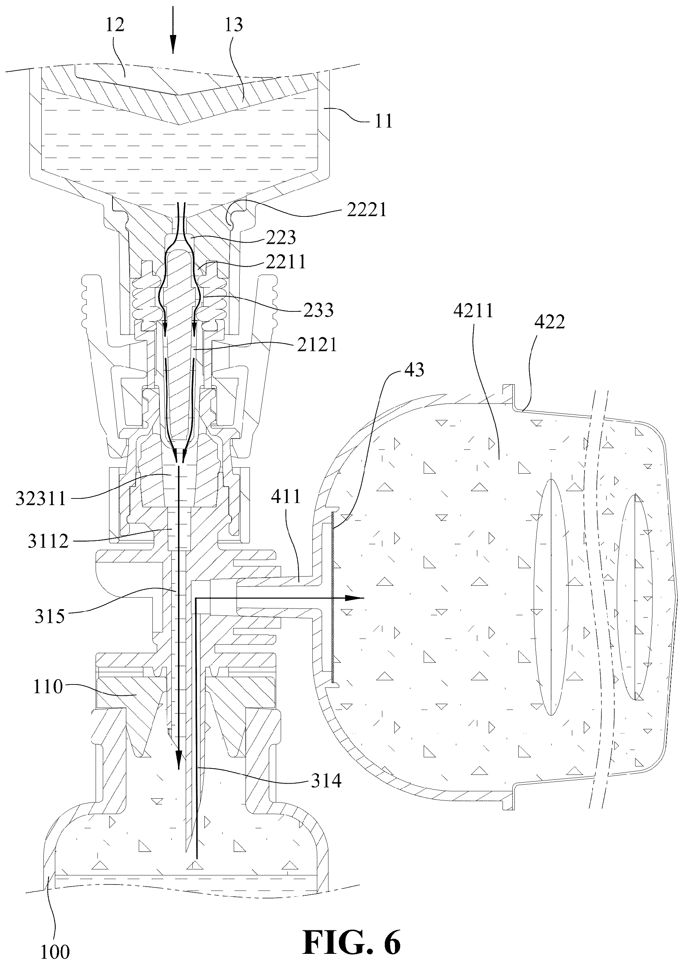

The drug-adding procedure of the first embodiment includes the following steps: As shown in FIG. 5, the fastener 313 fastens a drug bottle 100, and the pointed tip 3121 of the puncture portion 312 punctures a stopper 110 of the drug bottle 100. In some embodiments, the fastener 313 fastens an infusion end (not shown) of a soft bag, and the pointed tip 3121 of the puncture portion 312 punctures a thin film (not shown) of the infusion end of the soft bag; As shown in FIG. 6, with the first embodiment on top and the drug bottle 100 or soft bag below, the plunger handle 121 is pushed, and the plunger 12 drives the piston 13 to move inward, so that the hazardous drug inside the syringe 11 is injected into the drug bottle 100 or soft bag from top to bottom sequentially through the connecting channel 223, groove 2211, perforations 233, first channel 2121, second channel 32311, shaft hole 3112, and second flow channel 315. At the same time, the toxic gas inside the drug bottle 100 or soft bag passes sequentially through the first flow channel 314 and tube body 411 from bottom to top, and smoothly passes through the hydrophobic and breathable membrane 43, and is then injected into the first chamber 4211. The air bag 422 will continue to expand. Therefore, during the drug-adding procedure, the first embodiment can collect toxic gases through the first chamber 4211, maintaining a pressure balance between the internal atmospheric pressure of the drug bottle 100 or soft bag and the external atmospheric pressure. This also prevents small amounts of hazardous drugs inside the drug bottle 100 or soft bag from passing through the needle-free connector 32 in gaseous form or by evaporation. No hazardous drug residue remains on the outer surface of the needle-free connector 32, and toxic gases will not diffuse into the external space through the needle-free connector 32. Medical personnel will not come into contact with hazardous drugs on their skin, nor will they inhale toxic gases into their lungs. Furthermore, the protruding ring 2221 prevents liquid or gas leakage from the gap between the upper sleeve portion 2111 and the first engaging portion 22, achieving a sealing effect.

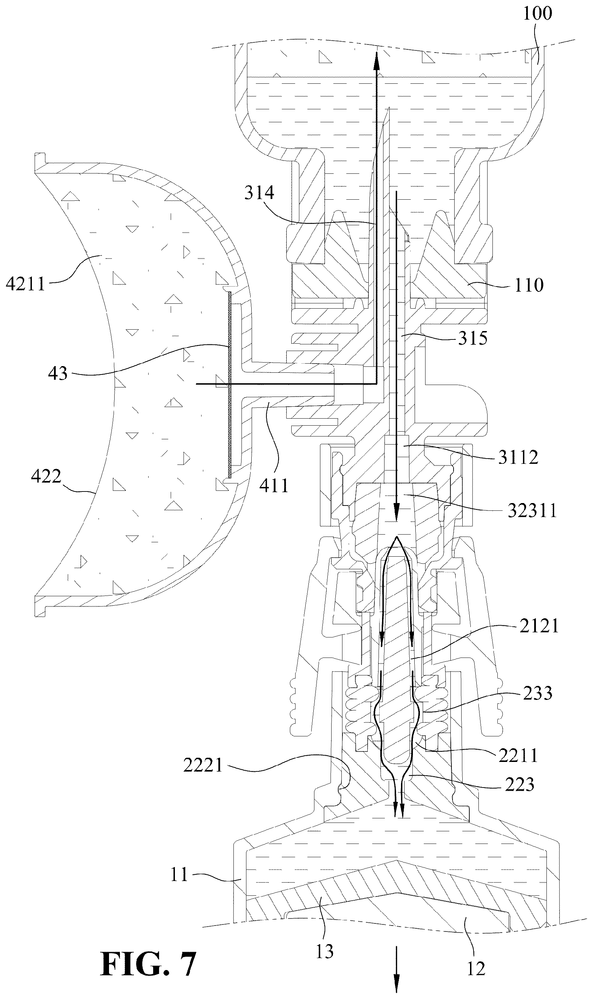

The drug-dispensing procedure in the first embodiment includes the following steps: pushing the plunger handle 121, the plunger 12 drives the piston 13 to move inward, the piston 13 will inject clean air from the inside of the syringe 11 into the first chamber 4211 sequentially through the first flow channel 314 and the inside of the tube body 411, and the air bag 422 will continue to inflate; as shown in FIG. 5, the fastener 313 fastens the drug bottle 100, and the pointed tip 3121 of the puncture portion 312 punctures the bottle stopper 110 of the drug bottle 100; in some embodiments, the fastener 313 fastens the infusion end of the soft bag (not shown), and the pointed tip 3121 of the puncture portion 312 punctures the thin film of the infusion end of the soft bag (not shown); as shown in FIG. 7, the drug bottle 100 or the soft bag on top and the first embodiment on the bottom, pulling the plunger handle 121 drives the piston 13 to move outward. This allows the hazardous drug inside the drug bottle or the soft bag to be injected into the syringe 11 from top to bottom sequentially through the second flow channel 315, shaft hole 3112, second channel 32311, first channel 2121, perforations 233, grooves 2211, and connecting channel 223. At the same time, clean air inside the first chamber 4211 passes smoothly through the hydrophobic and breathable membrane 43 and is injected into the drug bottle 100 or the soft bag from bottom to top through the tube body 411 and the first flow channel 314. The air bladder 422 continuously contracts. Therefore, during the drug-dispensing procedure, the first embodiment can collect and provide clean air through the first chamber 4211, maintaining a pressure balance between the atmospheric pressure inside the drug bottle 100 or the soft bag and the external atmospheric pressure, making it easier to remove the hazardous drug from the drug bottle 100 or the soft bag. Furthermore, the protruding ring 2221 can prevent liquid or gas from leaking through the gap between the upper sleeve portion 2111 and the first engaging portion 22, achieving a sealing effect.

Through the above-described drug-adding and dispensing procedures, hazardous drugs will not enter the first chamber 4211 through the inside of the first flow channel 314 and the tube body 411. However, some uninformed medical personnel may place the first embodiment at the bottom and the drug bottle 100 or soft bag at the top during drug-adding procedure, causing the hazardous drugs inside the drug bottle 100 or soft bag to easily pass through the inside of the first flow channel 314 and the tube body 411 from top to bottom. In this case, the hazardous drugs will be blocked by the hydrophobic and breathable membrane 43 and cannot pass through it, thus preventing the hazardous drugs from entering the first chamber 4211. In addition, the hydrophobic and breathable membrane 43 has the advantages of simple structure, small size, low cost, and resistance to damage.

During drug-dispensing, the outer surface of the conduit 212 may come into contact with hazardous drugs. As shown in FIG. 4, when the first connector 20 separates from the needle-free connector 32, the second sealing portion 3232 can completely scrape away the hazardous drug from the outer surface of the conduit 212. Therefore, after the conduit 212 is detached from the second sealing portion 3232, there is no hazardous drug residue on the outer surface of the conduit 212, preventing hazardous drug residue from staying on the outer surface of the conduit 212 and ensuring that medical personnel's skin does not come into contact with the hazardous drug.

The initial air volume of the airbag 422 is preset to the midpoint between the contraction limit and the expansion limit, and is not preset at the contraction limit. This allows the user to directly perform drug-adding or drug-dispensing actions. The airbag has a large air storage space, allowing for multiple runs of drug-adding or drug-dispensing without the problem of the airbag expanding or contracting to its limit.

Preferably, the second elastic valve 323 is made of silicone or rubber. As shown in FIGS. 3A and 3B, after the conduit 212 is opened and passes through the slit 32321 (see FIG. 4), the second sealing portion 3232 can tightly cover one end of the conduit 212, preventing hazardous drugs and toxic gases from leaking from the slit 32321 during drug-dispensing. As shown in FIG. 4, after the conduit 212 is disengaged from the second sealing portion 3232, the slit 32321 quickly returns to its initial closed state, preventing the conduit 212 from being exposed and preventing hazardous drugs and toxic gases from leaking from the slit 32321.

In some embodiments, the fastener 313 has two extensions (not shown) on each side. The user's hand can easily pry open the fastener using these extensions and align the puncture portion 312 with the stopper 110.

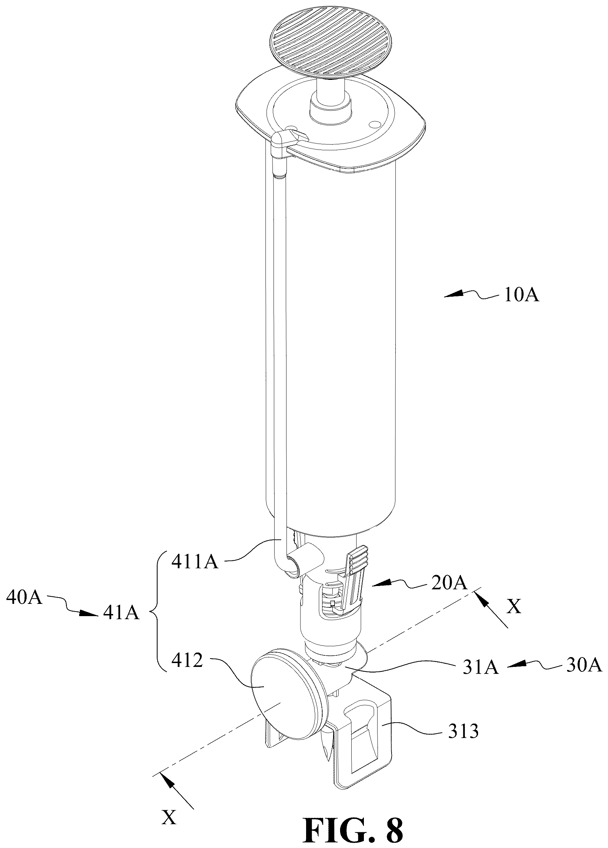

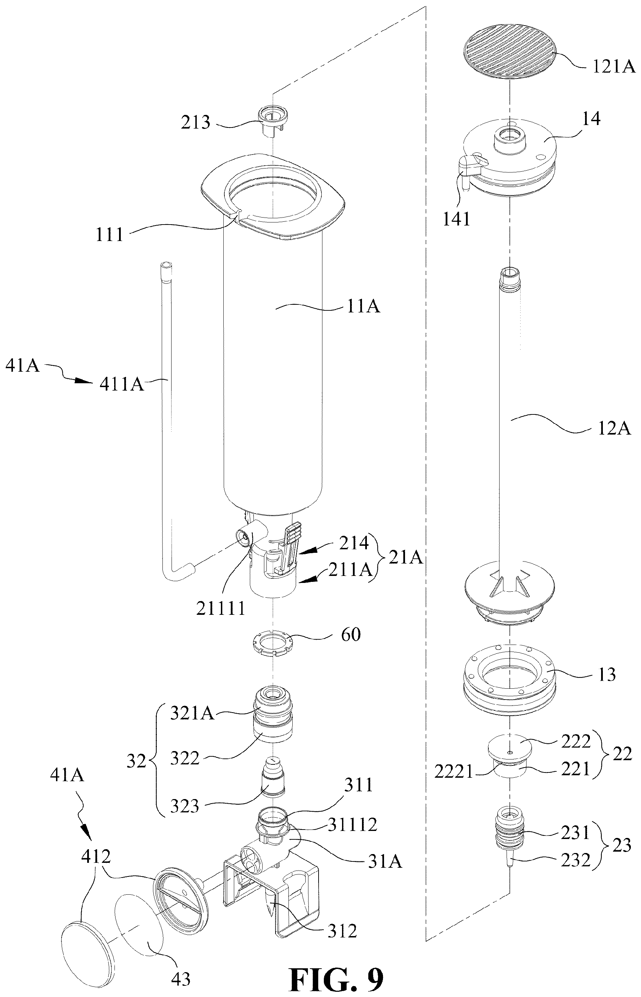

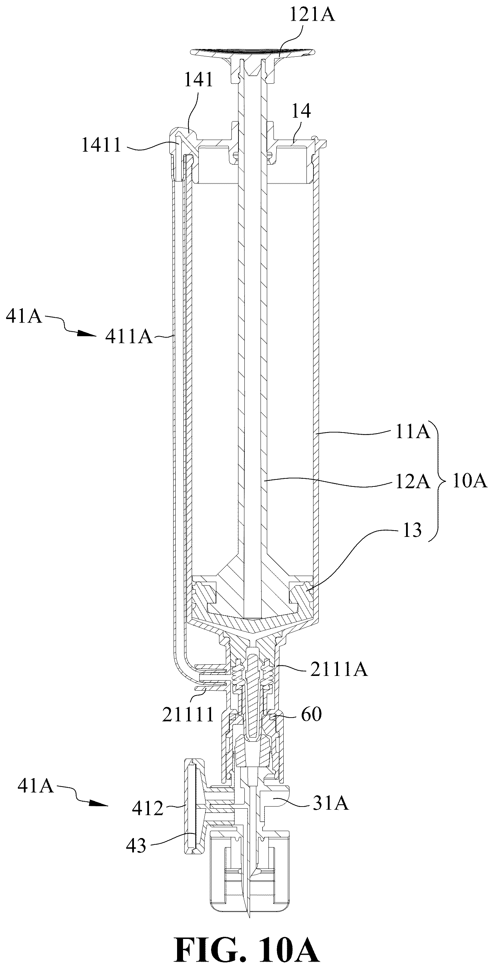

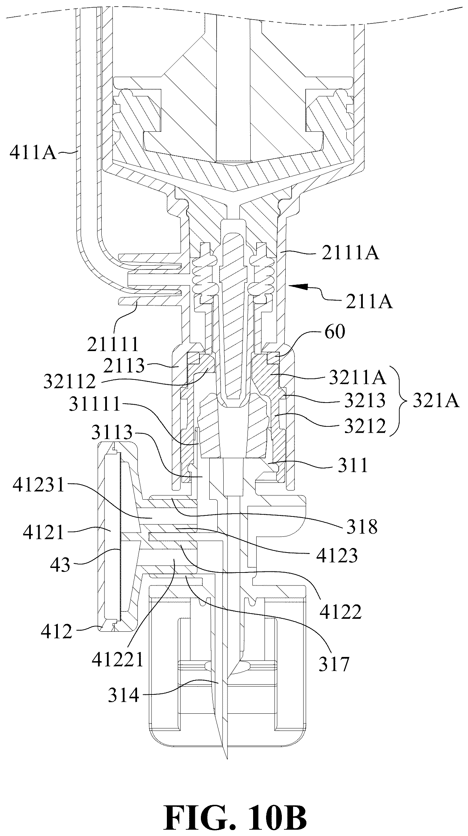

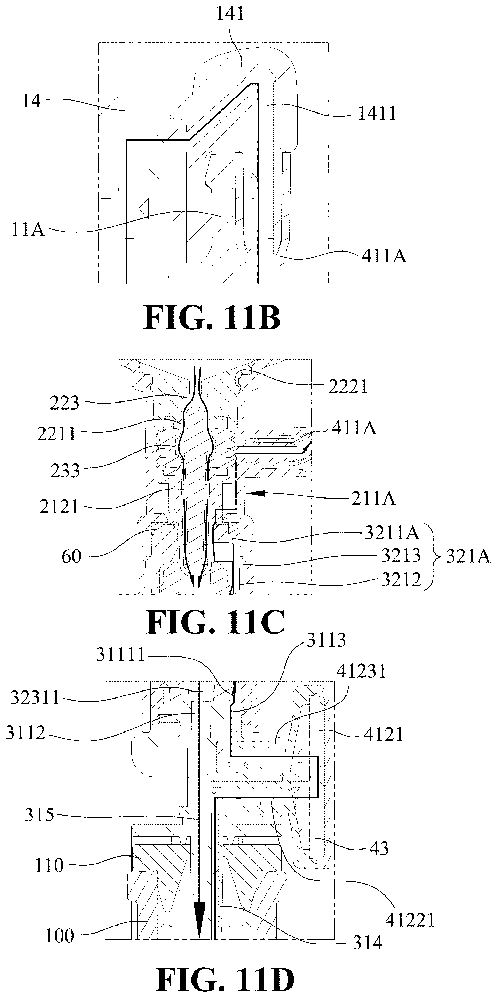

As shown in FIGS. 8, 9, 10A, and 10B, the difference between the second embodiment and the first embodiment is as follows: Firs, the syringe 10A further includes a cover 14, which is snap-fitted onto the top of the syringe 11A, and the outer side wall of the cover 14 and the inner side wall of the syringe 11A are fully fitted together to achieve a sealing effect (see FIG. 11B). One side of the cover 14 has a second protruding portion 141, which is disposed with an air passage 1411. An embedding groove 111 is provided on one side of the top of the syringe 11A, and the second protruding portion 141 is fixed in the embedding groove 111. The air passage 1411 communicates with the inside of the syringe 11A, so that the air passage 1411 and the inside of the syringe 11A together form a collecting portion. Second, the plunger 12A passes through the cover 14, and the plunger handle 121A is detachably mounted on the top end of plunger 12A and located above cover 14; third, the inner diameter of the first end of the upper housing 3211A of housing 321A is larger than the outer diameter of conduit 212; fourth, the top end of tube body 411A is connected to the second protruding portion 141, and the inside of tube body 411A communicates with air passage 1411; fifth, one side of the upper sleeve portion 2111A of sleeve 211A of body 21A has a third protruding portion 21111, the bottom end of tube body 411A is connected to the third protruding portion 21111, and the inside of tube body 411A communicates with the inside of sleeve 211A; sixth, one side of the base 31A of second connector 30A has a first insertion portion 317 and a second insertion portion 318, and the guide portion 41A of pressure balancing device 40A further includes a filter 412, the filter 412 having a second chamber 4121. An inlet end 4122 and an outlet end 4123 are provided. The inlet end 4122 is inserted into the first insertion portion 317 and has an inlet 41221. The outlet end 4123 is inserted into the second insertion portion 318 and has an outlet 41231. The second chamber 4121 communicates with the inlet 41221 and the outlet 41231. The inlet 41221 communicates with the first flow channel 314. The assembly portion 311A has a third flow channel 3113. The outlet 41231 communicates with the third flow channel 3113. The inner sidewall of the assembly groove 3111 has a fourth flow channel 31111. The fourth flow channel 31111 communicates with the third flow channel 3113. A hydrophobic and breathable membrane 43 is disposed in the second chamber 4121. The hazardous drug liquid-gas exchange device further includes a sealing ring 60, which is disposed on the top outer side of the upper housing 3211A and abuts against the inner side of the lower sleeve portion 2113. That is, the sealing ring 60 is part of the second connector 30. In some embodiments, the sealing ring 60 is disposed on the inner side of the lower sleeve portion 2113 and abuts against the top outer side of the upper housing 3211A, i.e., the sealing ring 60 is part of the first connector; eighth, when the first connector 20A is mated with the needle-free connector 32, the inside of the sleeve 211A communicates with the second opening 32111, the second opening 32111 communicates with the inside of the housing 321A, and the inside of the housing 321A communicates with the fourth flow channel 31111.

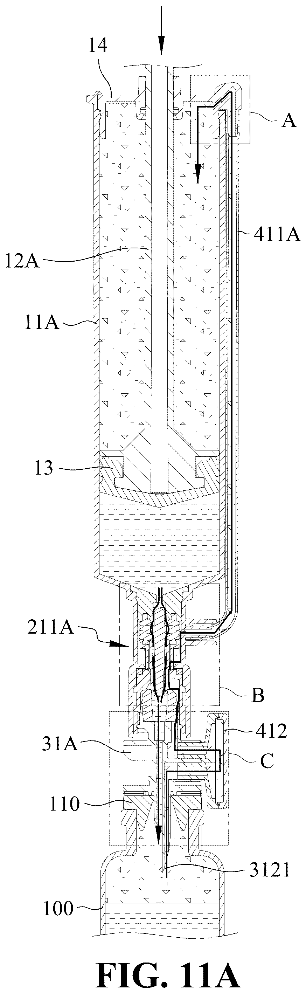

The drug-adding procedure of the second embodiment includes the following steps: as shown in FIGS. 11A and 11D, the pointed tip 3121 of the puncture portion 312 punctures the stopper 110 of the drug bottle 100; in some embodiments, the pointed tip 3121 of the puncture portion 312 punctures the thin film of the infusion end of the soft bag. As shown in FIGS. 11A, 11B, 11C and 11D, with the second embodiment on top and the drug bottle 100 or soft bag at the bottom, the plunger handle 121A is pushed, and the plunger 12A drives the piston 13 to move inward, so that the hazardous drug inside the syringe 11A passes from top to bottom sequentially through the connecting channel 223, the groove 2211, the perforations 233, the first channel 2121, the second channel 32, the shaft hole 3112, and the second flow channel 315 to enter the inside of the drug bottle 100 or soft bag. At the same time, the toxic gas inside the drug bottle 100 or soft bag passes sequentially through the first flow channel 314 and the inlet 41221 from bottom to top and passes smoothly through the hydrophobic and breathable membrane 43 to be injected into the second chamber 4121. The toxic gas in the second chamber 4121 passes smoothly through the hydrophobic and breathable membrane 43 and passes sequentially through the outlet 41231, the third flow channel 3113, the fourth flow channel 31111, the inside of the housing 321A, the inside of the sleeve 211A, the inside of the tube body 411A and the air passage 1411 from bottom to top and is injected into the inside of the syringe 11A. Therefore, during the drug-adding procedure, the second embodiment can collect toxic gases inside the syringe 11A, maintaining a pressure balance between the internal atmospheric pressure of the drug bottle 100 or soft bag and the external atmospheric pressure. This also prevents small amounts of hazardous drugs inside the drug bottle 100 or soft bag from passing through the needle-free connector 32 in gaseous form or by evaporation. No hazardous drugs remain on the outer surface of the needle-free connector 32, and toxic gases will not diffuse into the external space through the needle-free connector 32. Medical personnel will not come into contact with hazardous drugs on their skin, nor will they inhale toxic gases into their lungs. Furthermore, the sealing ring 60 prevents hazardous drugs and toxic gases from leaking into the external space through the gap between the inner side of the lower sleeve portion 2113 and the outer side of the upper housing 3211A. Medical personnel will not come into contact with hazardous drugs on their skin, nor will they inhale toxic gases into their lungs.

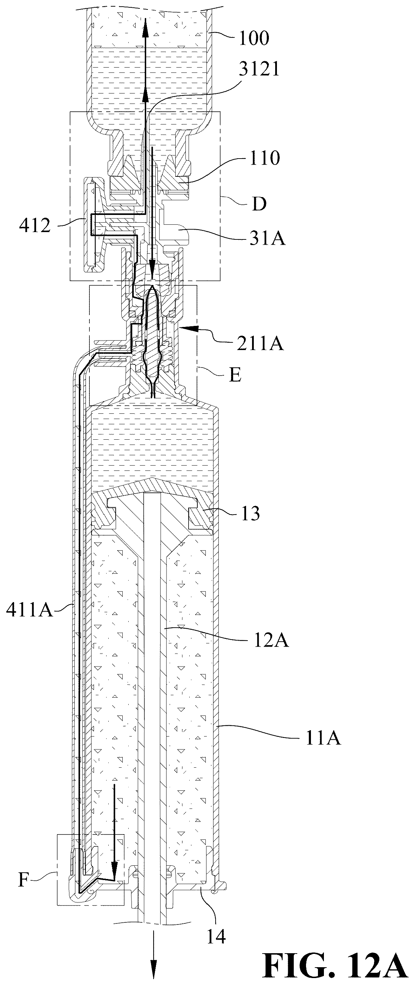

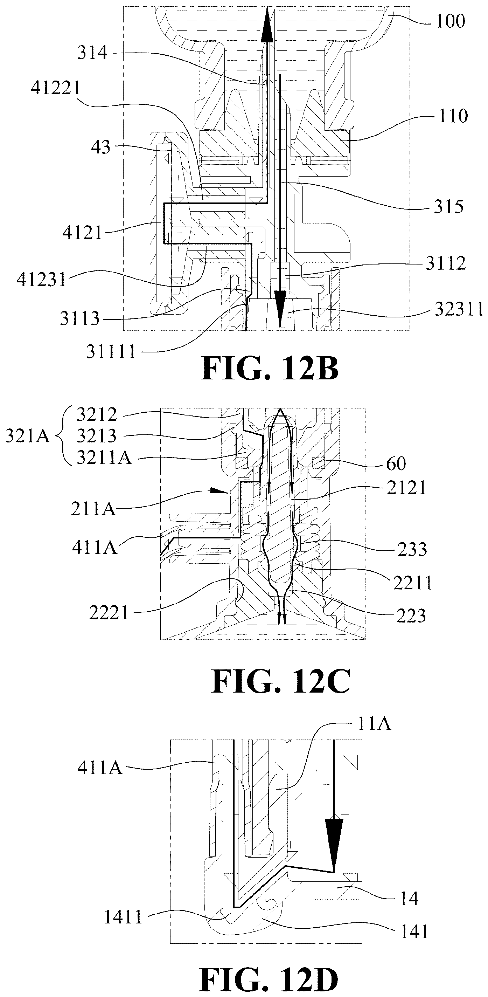

The drug-dispensing procedure of the second embodiment includes the following steps: pushing the plunger handle 121A, the plunger 12A drives the piston 13 to move inward, clean air from the external space passes sequentially through the first flow channel 314 and the inlet 41221 and smoothly passes through the hydrophobic and breathable membrane 43, and then is injected into the second chamber 4121. The clean air in the second chamber 4121 smoothly passes through the hydrophobic and breathable membrane 43, and then passes sequentially through the outlet 41231, the third flow channel 3113, and the fourth flow channel. 31111, the inside of the housing 321A, the inside of the sleeve 211A, the inside of the tube body 411A, and the air passage 1411, and then to be injected into the inside of the syringe 11A; as shown in FIGS. 12A and 12D, the pointed tip 3121 of the puncture portion 312 punctures the stopper 110 of the drug bottle 100; in some embodiments, the pointed tip 3121 of the puncture portion 312 punctures the thin film of the infusion end of the soft bag. As shown in FIGS. 12A, 12B, 12C, and 12D, with the drug bottle 100 or the soft bag on top and the second embodiment below, pulling the plunger handle 121A causes the plunger 12 to drive the piston 13 to move outward. This allows the hazardous medicine inside the drug bottle 100 or soft bag to be injected into the syringe 11A from top to bottom sequentially through the second flow channel 315, shaft hole 3112, second channel 32311, first channel 2121, perforations 233, grooves 2211, and connecting channel 223. At the same time, clean air inside the syringe 11A passes through the air passage 1411 from bottom to top, the inside of the tube body 411A, the inside of the sleeve 211A, the inside of the housing 321A, the fourth flow channel 31111, the third flow channel 3113, and the outlet 41231, and then smoothly through the hydrophobic and breathable membrane 43, and then being injected into the second chamber 4121. The clean air in the second chamber 4121 smoothly passes through the hydrophobic and breathable membrane 43, and then sequentially from bottom to top through the inlet 41221 and the first flow channel 314 into the inside of the drug bottle 100 or soft bag. Therefore, in the second embodiment, during the drug-dispensing procedure, clean air can be collected and supplied through the inside of the syringe 11A, allowing the atmospheric pressure inside the drug bottle 100 or soft bag to maintain a pressure balance with the external atmospheric pressure, and making it easier to remove hazardous drugs from the inside of the drug bottle 100 or soft bag. Furthermore, the sealing ring 60 prevents hazardous drugs and clean air from leaking out into the external space through the gap between the inner side of the lower sleeve portion 2113 and the outer side of the upper housing 3211A.

Through the above-described correct drug-adding and drug-dispensing procedures, hazardous drugs will not enter the second chamber 4121 through the first flow channel 314 and inlet 41221. However, some uninformed medical personnel may place the second embodiment at the bottom and the drug bottle 100 or soft bag at the top during the drug-dispensing procedure, causing the hazardous drugs inside the drug bottle 100 or soft bag to easily pass through the first flow channel 314 and inlet 41221 sequentially from top to bottom. In this case, the hazardous drugs will be blocked by the hydrophobic and breathable membrane 43, thus preventing the hazardous drugs from entering the second chamber 4121.

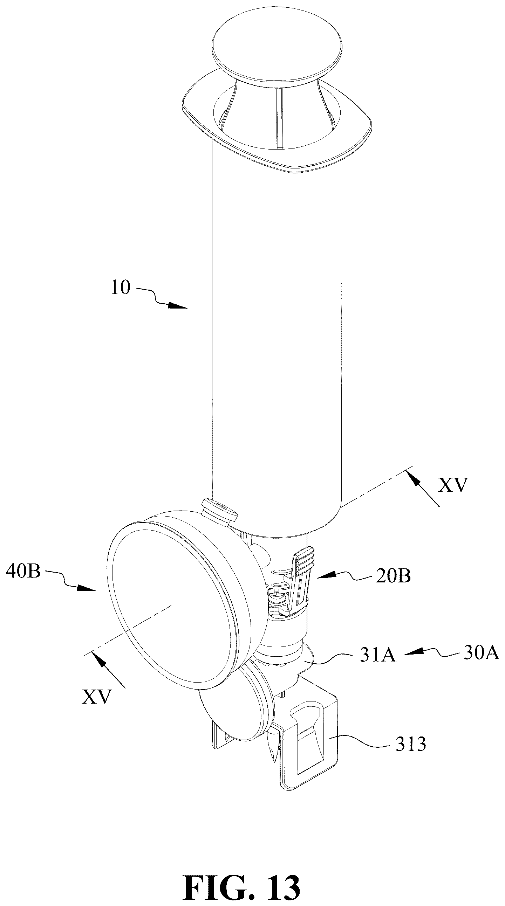

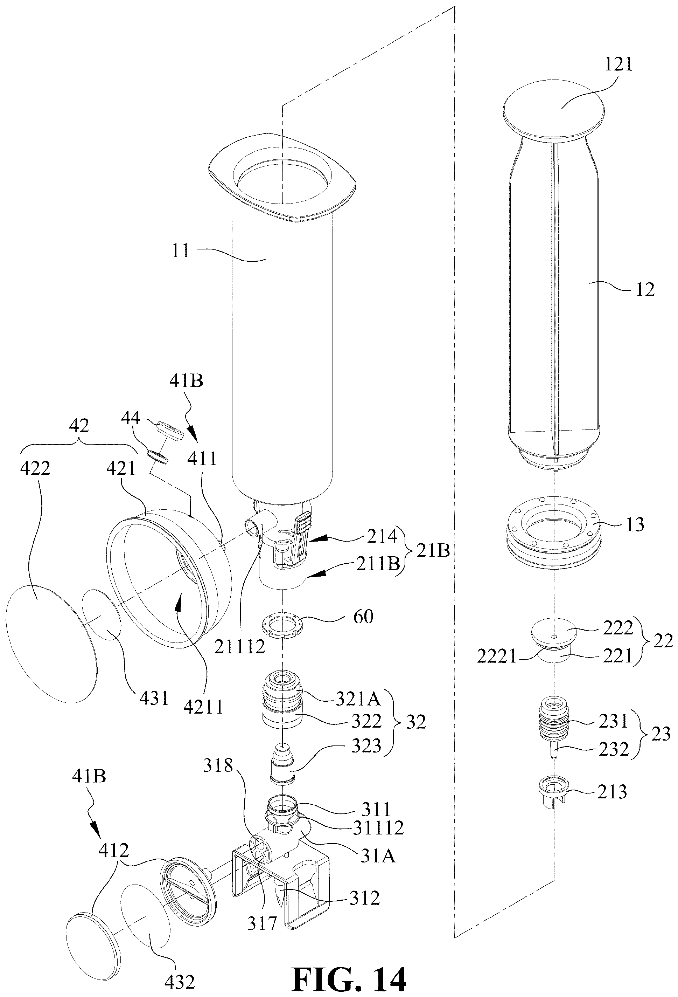

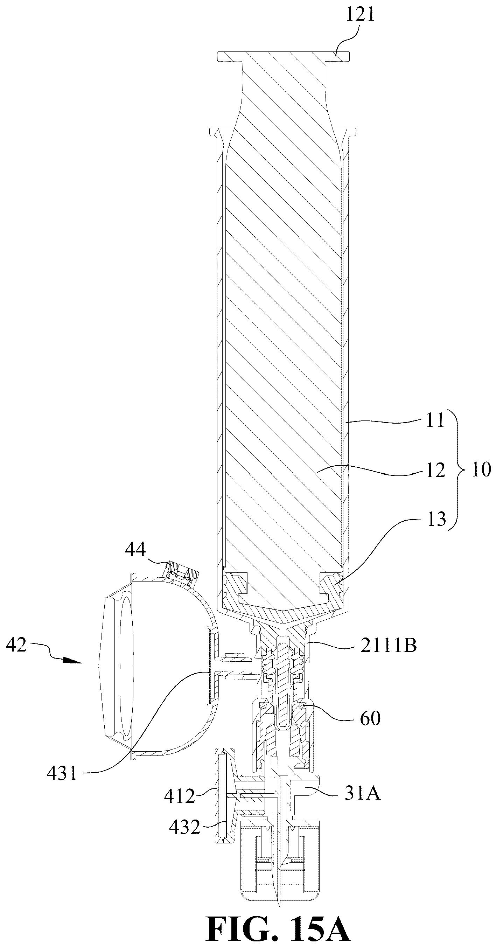

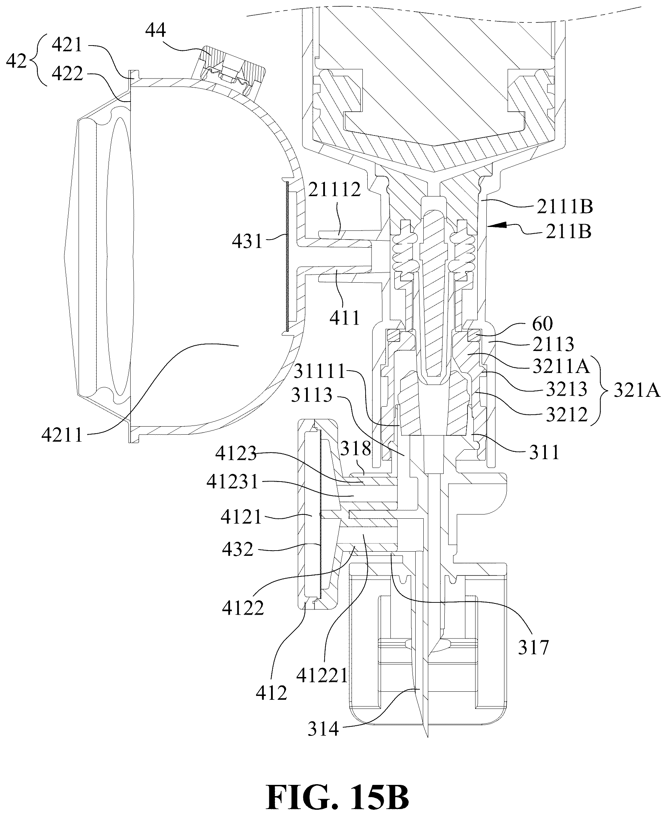

As shown in FIGS. 13, 14, 15A, and 15B, the differences between the third embodiment and the first embodiment are as follows: First, the pressure balancing device 40B includes a first hydrophobic and breathable membrane 431, a second hydrophobic and breathable membrane 432, and a check valve 44. The check valve 44 is disposed on an outer cover 421 (at any position on the outer cover 421, whether inside or outside the outer cover 421) to allow clean air from the external space to enter the first chamber 4211 and to prevent air from flowing out of the first chamber 4211. Second, the collecting portion 42 includes the outer cover 421 and an airbag 422. The outer cover 421 is integrally formed with one end of the tube body 411, and the outer cover 421 has a first chamber 4211. The inside of the tube body 411 communicates with the first chamber 4211. The first hydrophobic and breathable membrane 431 is disposed in the first chamber 4211 and close to the opening of the tube body 411. The airbag 422 is disposed at the opening of the first chamber 4211. Third, the upper sleeve portion 2111B has a fourth protruding portion 21112 on one side. The base 31A of the second connector 30A has a first insertion portion 317 and a second insertion portion 318 on one side. The guide portion 41B of the pressure balancing device 40B further includes a tube body 411 and a filter 412. The tube body 411 is connected to the fourth protruding portion 21112. The inside of the tube body 411 communicates with the inside of the sleeve 211B. The filter 412 has a second chamber 4121 and is disposed with an inlet end 4122 and an outlet end 4123. The inlet end 4122 is inserted into the first insertion portion 317 and has an inlet 41221. The outlet end 4123 is inserted into the second insertion portion 318 and has an outlet 41231. The second chamber 4121 communicates with the inlet 41221 and the outlet 41231. The inlet 41221 communicates with the first flow channel 314. The assembly portion 311A has a third flow channel 3113. The outlet 41231 communicates with the third flow channel 3113. The inner sidewall of the assembly groove 3111 has a fourth flow channel 31111. The fourth flow channel 31111 communicates with the third flow channel 3113. A second hydrophobic and breathable membrane 432 is disposed in the second chamber 4121. Fourth, the hazardous drug liquid-gas exchange device further includes a sealing ring 60, which is disposed on the top outer side of the upper housing 3211A and abuts against the inner side of the lower sleeve portion 2113. That is, the sealing ring 60 is part of the second connector 30. In some embodiments, the sealing ring 60 is disposed on the inner side of the lower sleeve portion 2113 and abuts against the top outer side of the upper housing 3211A. That is, the sealing ring 60 is part of the first connector. Fifth, when the first connector 20B is mated with the needle-free connector 32, the inside of the sleeve 211B communicates with the second opening 32111, the second opening 32111 communicates with the inside of the housing 321A, and the inside of the housing 321A communicates with the fourth flow channel 31111.

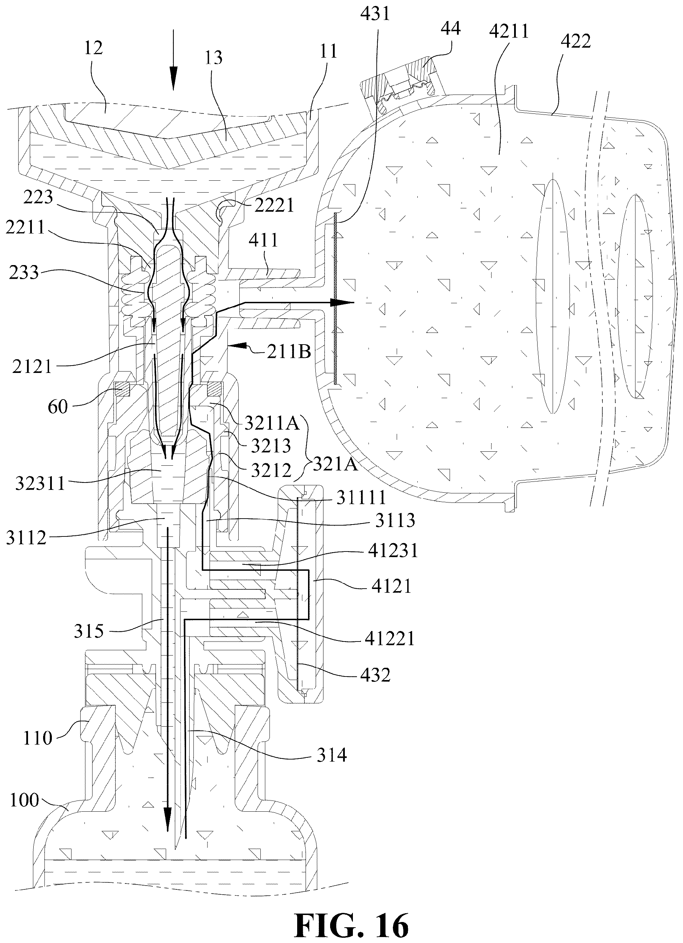

The drug-adding procedure of the third embodiment includes the following steps: as shown in FIG. 16, the pointed tip 3121 of the puncture portion 312 punctures the stopper 110 of the drug bottle 100; in some embodiments, the pointed tip 3121 of the puncture portion 312 punctures the thin film of the infusion end of the soft bag. As shown in FIG. 16, with the third embodiment on top and the drug bottle 100 or soft bag at the bottom, the plunger handle 121 is pushed, and the plunger 12 drives the piston 13 to move inward, so that the hazardous drug inside the syringe 11 passes from top to bottom sequentially through the connecting channel 223, the groove 2211, the perforations 233, the first channel 2121, the second channel 32, the shaft hole 3112, and the second flow channel 315 to enter the inside of the drug bottle 100 or soft bag. At the same time, the toxic gas inside the drug bottle 100 or soft bag passes sequentially through the first flow channel 314 and the inlet 41221 from bottom to top and passes smoothly through the second hydrophobic and breathable membrane 432 to be injected into the second chamber 4121. The toxic gas in the second chamber 4121 passes smoothly through the second hydrophobic and breathable membrane 432 and passes sequentially through the outlet 41231, the third flow channel 3113, the fourth flow channel 31111, the inside of the housing 321A, the second opening 32111, the inside of the sleeve 211B, the inside of the tube body 411, and then passes smoothly through the first hydrophobic and breathable membrane 431, and is injected into the first chamber 4211, while the airbag 422 continues to inflate. Therefore, during the drug-adding procedure, the third embodiment can collect toxic gases inside the first chamber 4211, maintaining a pressure balance between the internal atmospheric pressure of the drug bottle 100 or soft bag and the external atmospheric pressure. This also prevents small amounts of hazardous drugs inside the drug bottle 100 or soft bag from passing through the needle-free connector 32 in gaseous form or by evaporation. No hazardous drugs remain on the outer surface of the needle-free connector 32, and toxic gases will not diffuse into the external space through the needle-free connector 32. Medical personnel will not come into contact with hazardous drugs on their skin, nor will they inhale toxic gases into their lungs. Furthermore, the sealing ring 60 prevents hazardous drugs and toxic gases from leaking into the external space through the gap between the inner side of the lower sleeve portion 2113 and the outer side of the upper housing 3211A. Medical personnel will not come into contact with hazardous drugs on their skin, nor will they inhale toxic gases into their lungs.

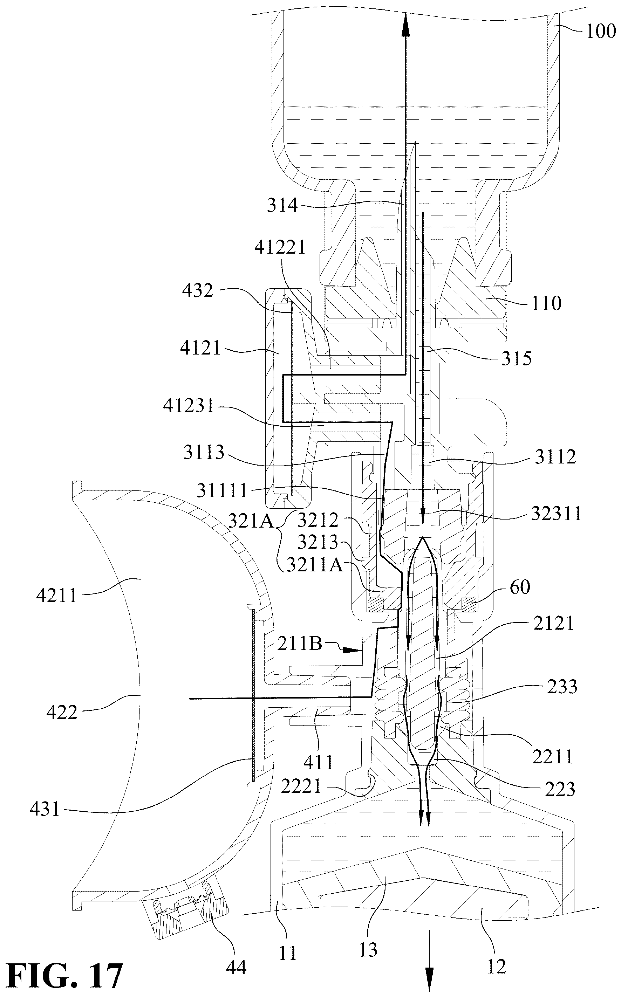

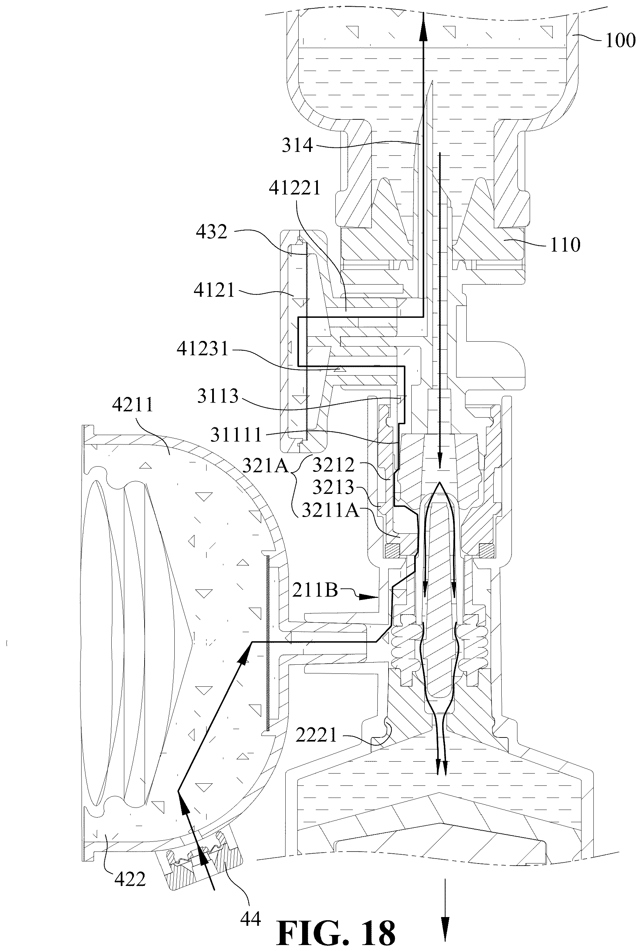

The drug-dispensing procedure of the third embodiment includes the following steps: pushing the plunger handle 121, the plunger 12 drives the piston 13 to move inward, clean air from the external space passes sequentially through the first flow channel 314 and the inlet 41221 and smoothly passes through the second hydrophobic and breathable membrane 432, and then is injected into the second chamber 4121. The clean air in the second chamber 4121 smoothly passes through the second hydrophobic and breathable membrane 432, and then passes sequentially through the outlet 41231, the third flow channel 3113, and the fourth flow channel. 31111, the inside of the housing 321A, the second opening 32111, the inside of the sleeve 211B, the inside of the tube body 411, passes smoothly through the first hydrophobic and breathable membrane 431, and then to be injected into the inside of the first chamber 4211, while the airbag continues to inflate; as shown in FIG. 17, with the drug bottle 100 or the soft bag on top and the third embodiment below, pulling the plunger handle 121 causes the plunger 12 to drive the piston 13 to move outward. This allows the hazardous medicine inside the drug bottle 100 or soft bag to be injected into the syringe 11 from top to bottom sequentially through the second flow chanfirst nel 315, the shaft hole 3112, the second channel 32311, the first channel 2121, the perforations 233, the grooves 2211, and the connecting channel 223. At the same time, the clean air inside the first chamber 4211, after passing smoothly through the first hydrophobic and breathable membrane 431, passes from bottom to top sequentially through the inside of the tube body 411, the inside of the sleeve 211B, the second opening 32111, the inside of the housing 321A, the fourth flow channel 31111, the third flow channel 3113, and the outlet 41231, and then smoothly through the hydrophobic and breathable membrane 431, and then being injected into the second chamber 4121. The clean air in the second chamber 4121 smoothly passes through the second hydrophobic and breathable membrane 432, and then sequentially from bottom to top through the inlet 41221 and the first flow channel 314 into the inside of the drug bottle 100 or soft bag, while the airbag 422 continues to inflate. As shown in FIG. 18, after the airbag 422 contracts, the clean air in the first chamber 4211 is exhausted, and the clean air from the external space passes through the check valve 44 to enter the first chamber 4211. The clean air inside the first chamber 4211 passes smoothly through the first hydrophobic and breathable membrane 431 and then passes from bottom to top through the inside of the tube body 411, the inside of the sleeve 211B, the second opening 32111, the inside of the housing 321A, the fourth flow channel 31111, the third flow channel 3113, and the outlet 41231, and passes smoothly through the second hydrophobic and breathable membrane 432, to be injected into the second chamber 4121. The clean air in the second chamber 4121 passes smoothly through the second hydrophobic and breathable membrane 432 and then passes from bottom to top through the inlet 41221 and the first flow channel 314 and is injected into the inside of the drug bottle 100 or soft bag. Therefore, in the third embodiment, during the drug-dispensing procedure, clean air can be collected and supplied through the inside of the first chamber 4211, and when the clean air from the first chamber is exhausted, the check valve provides the clean air, thus, allowing the atmospheric pressure inside the drug bottle 100 or soft bag to maintain a pressure balance with the external atmospheric pressure, and making it easier to remove hazardous drugs from the inside of the drug bottle 100 or soft bag. Furthermore, the sealing ring 60 prevents hazardous drugs and clean air from leaking out into the external space through the gap between the inner side of the lower sleeve portion 2113 and the outer side of the upper housing 3211A.

Through the above-described correct drug-adding and drug-dispensing procedures, the hazardous drug will not enter the second chamber 4121 through the first flow channel 314 and the inlet 41221, nor will it enter the first chamber 4211 through the inside of the tube body 411. However, some uninformed medical personnel may place the third embodiment at the bottom and the drug bottle 100 or soft bag at the top during drug-adding procedure, which causes the hazardous drugs inside the drug bottle 100 or soft bag to easily pass through the first flow channel 314 and the inlet 41221 sequentially from top to bottom. In this case, the hazardous drugs are blocked by the second hydrophobic and breathable membrane 432, preventing them from entering the second chamber 4121. Even if a small amount of hazardous drugs passes through the second hydrophobic and breathable membrane 432 and passes sequentially through the outlet 41231, the third flow channel 3113, the fourth flow channel 31111, the inside of the housing 321A, the second opening 32111, the inside of the sleeve 211B, and the inside of the tube body 411, this small amount of hazardous drugs will be blocked by the first hydrophobic and breathable membrane 431, preventing them from entering the first chamber 4211.

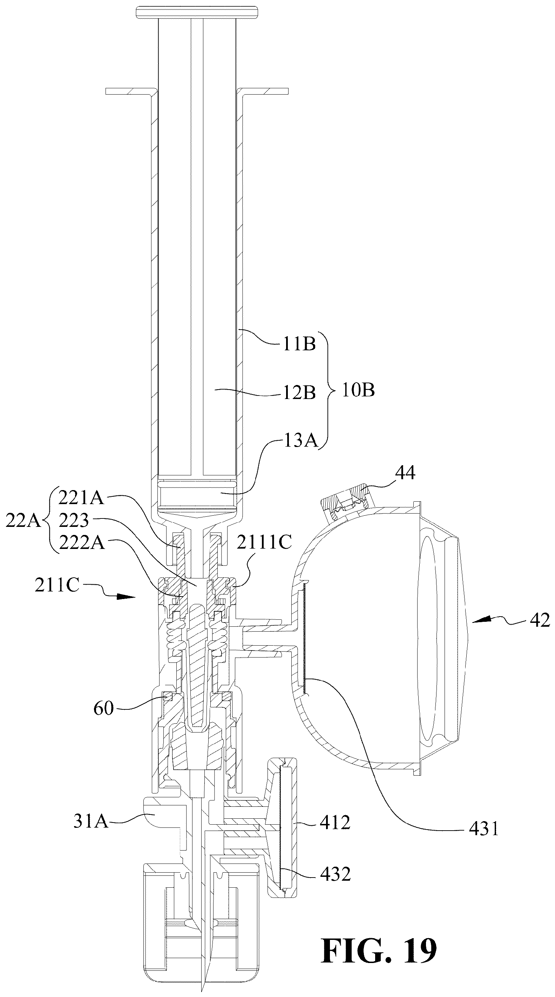

As shown in FIG. 19, the differences between the fourth embodiment and the third embodiment are as follows: First, the shapes of the syringe barrel 11B, the plunger 12B, and the piston 13A of the syringe 10B are slightly modified, and the bottom end of the syringe barrel 11B is a male Luer connector. Second, the upper sleeve portion 2111C of the sleeve 211C is separated from the bottom end of the syringe barrel 11B. Third, the first end 221A of the first engaging portion 22A is separated from the second end 222A, and the first end 221A is a female Luer connector, which is inserted into the bottom end of the syringe barrel 11B. In other words, the first end 221A can connect to syringes 11B of different volume capacities.

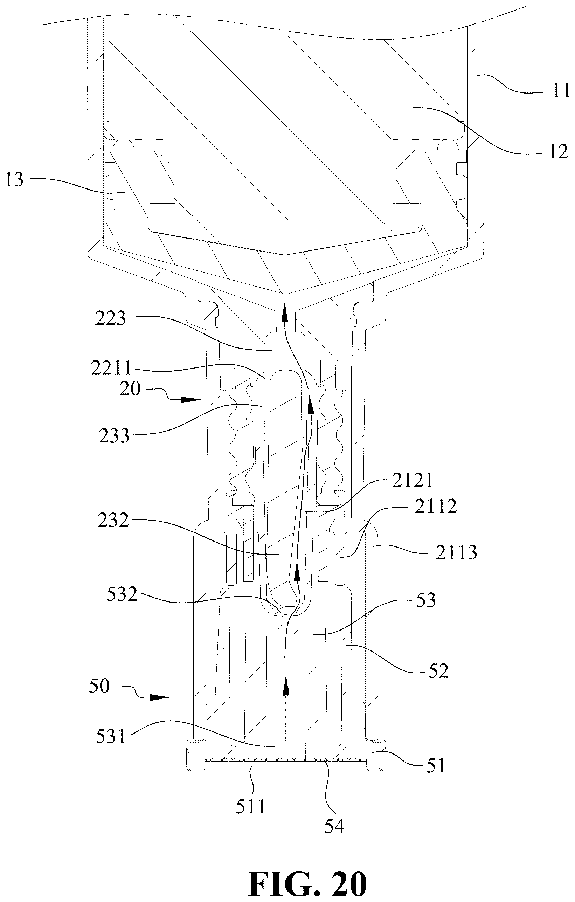

As shown in FIG. 20, the difference between the fifth embodiment and the first embodiment is that the hazardous drug liquid-gas exchange device of the present invention further includes a filter air guide cover 50. The filter air guide cover 50 includes a base 51, an outer ring wall 52, an inner ring wall 53, and a filter paper 54. The base 51 has a receiving groove 511. The outer ring wall 52 and the inner ring wall 53 are disposed on the base 51. The inner ring wall 53 is located inside the outer ring wall 52. The height of the outer ring wall 52 is higher than the height of the inner ring wall 53. The inner ring wall 53 is disposed with a air guide hole 531. A pushing portion 532 is disposed protrudingly from the top of the inner ring wall 53. The air guide hole 531 penetrates the pushing portion 532 and the base 51 and communicates with the receiving groove 511. The filter paper 54 is disposed in the receiving groove 511. When the first connector 20 aligns with the filter air guide cover 50, the base 51 abuts against the lower sleeve portion 2113, the outer ring wall 52 abuts against the middle sleeve portion 2112, and the pushing portion 532 pushes the first sealing portion 232 to bend and deform, causing the bottom end of the first sealing portion 232 to disengage from the first opening 2122, and the first channel 2121 communicates with the air guide hole 531. The drug-dispensing procedure of the fifth embodiment includes the following steps beforehand: pulling the plunger handle 121, the plunger 12 drives the piston 13 to move outward, and clean air from the external space sequentially passes through the filter paper 54, the air guide hole 531, the first channel 2121, the perforations 233, the groove 2211, and the connecting channel 223 before being injected into the inside of the syringe 11. Thus, in the fifth embodiment, clean air is prepared inside the syringe 11 before drug-dispensing. Furthermore, the inside of the syringe 11 communicates with the external space through the filter air guide cover 50 to achieve a sterilization effect. When the first connector 20 separates from the filter air guide cover 50, the first sealing portion 232 returns to its original state and closes the first opening 2122.

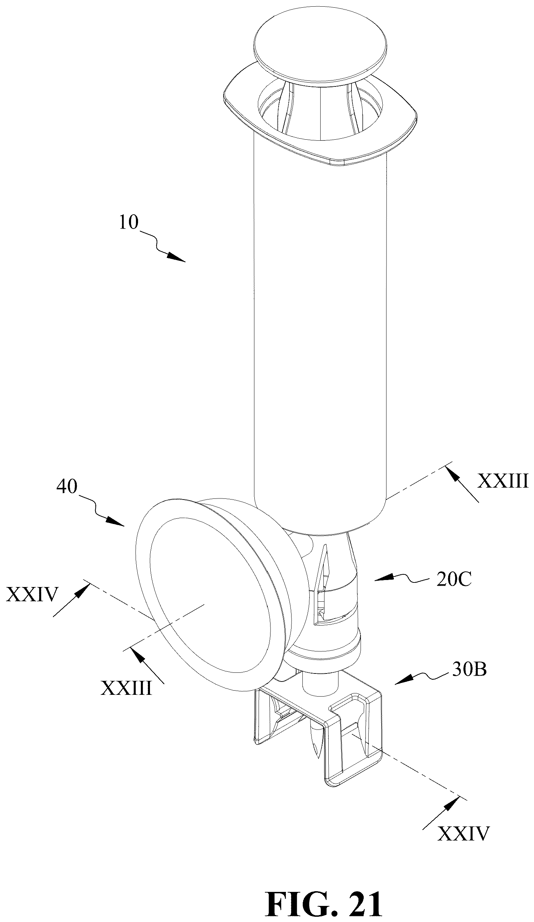

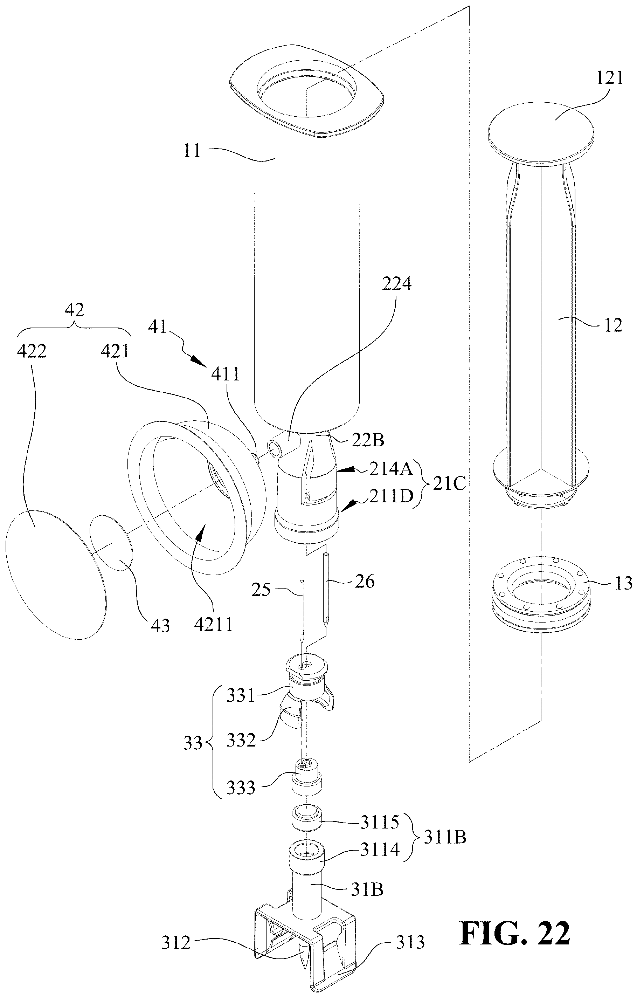

As shown in FIGS. 21 to 24, the differences between the sixth embodiment and the first embodiment are as follows: first, the first connector 20C includes a body 21C, a first engaging portion 22B, a first needle 25, and a second needle 26. The body 21C includes a sleeve 211D and two first snap-fit portions 214A. The sleeve 211D is disposed with two first through holes 2114A. The first snap-fit portions 214A are respectively located in the first through holes 2114A. Each first snap-fit portion 214A includes an arm portion 2141A, a connecting portion 2142A, and a hook portion 2143A. The connecting portion 2142A is disposed at the first engaging portion 22B and connects to the arm portion 2141A. The hook portion 2143A is disposed at one end of the arm portion 2141A, the first engaging portion 22B is integrally formed with the top end of the sleeve 211D and the bottom end of the syringe 11. The first engaging portion 22B has a first protruding portion 224. The first needle 25 and the second needle 26 are inserted into the first engaging portion 22B. The second needle 26 communicates with the inside of the syringe 11. Second, the second connector 30B includes a base 31B and a needle connector 33. The base 31B includes an assembly portion 311B, a puncture portion 312 and a fastener 313, and has a first flow channel 314 and a second flow channel 315. The first flow channel 314 surrounds the outside of the second flow channel 315. The assembly portion 311B includes a mounting base 3114 and a first protruding portion 224. A first stop block 3115 is disposed inside the mounting base 3114 and closes the openings of the first flow channel 314 and the second flow channel 315. The bottom end of the puncture portion 312 has a pointed tip 3121. The first flow channel 314 penetrates the bottom end of the puncture portion 312, and the second flow channel 315 also penetrates the bottom end of the puncture portion 312. A partition wall 3122 is provided between the first flow channel 314 and the second flow channel 315. The pointed tip 3121 is located at the center of the bottom end of the partition wall 3122. A fastener 313 is disposed on the outside of the puncture portion 312. The needle connector 33 includes a housing 331, two second engaging portions 332, and a second stop block 333. The housing 331 has a second snap-fit portion 3311, the second snap-fit portion 3311 is a ring protrusion and is disposed on the outer side of the top end of the housing 331. Each second snap-fit portion 332 includes an arm portion 3321, a connecting portion 3322, and a hook portion 3323. The connecting portion 3322 is disposed at the bottom end of the housing 331 and connects to the arm portion 3321. The hook portion 3323 is disposed at one end of the arm portion 3321. The second stop block 333 is disposed inside the housing 331. The mounting base 3114 is fixed to the hook portions 3323 of the second snap-fit portions 332. Third, the guide portion 41 includes a tube body 411, the tube body 411 is connected to the first protruding portion 224, and the inside of the tube body 411 communicates with the inside of the first needle 25.

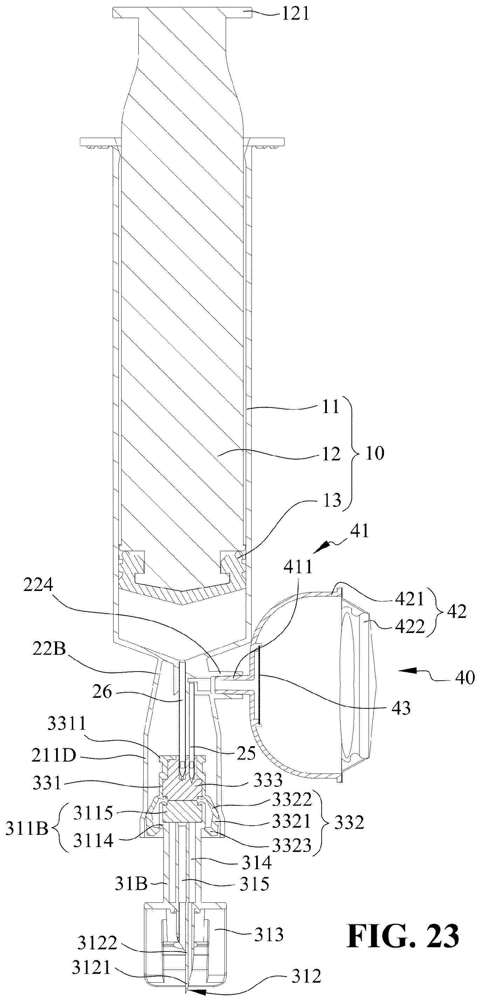

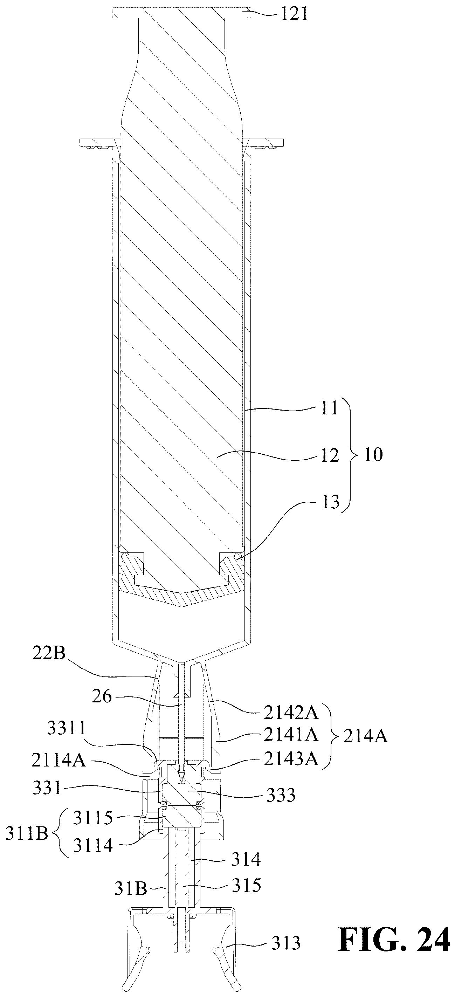

As shown in FIGS. 23 and 24, when the first connector 20C is mated with the needle connector 33, the hook portions 2143A of the first snap-fit portions 214A are fixed to the second snap-fit portion 3311, and the first needle 25 and the second needle 26 are inserted into the second stop block 333 but do not penetrate the second stop block 333.

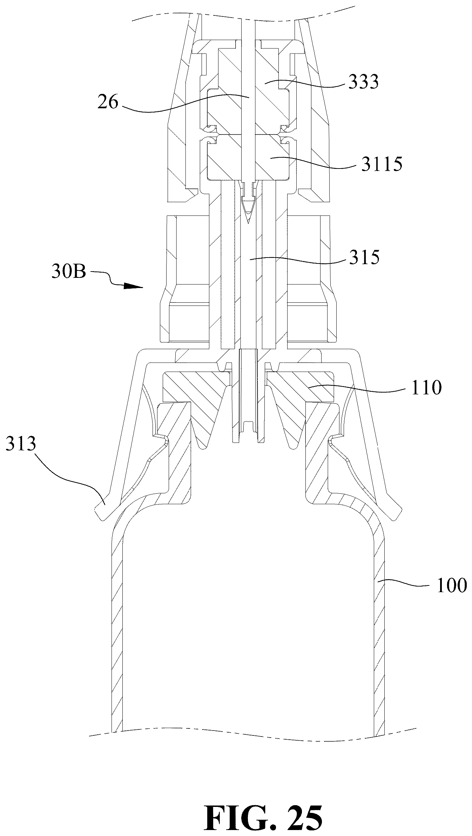

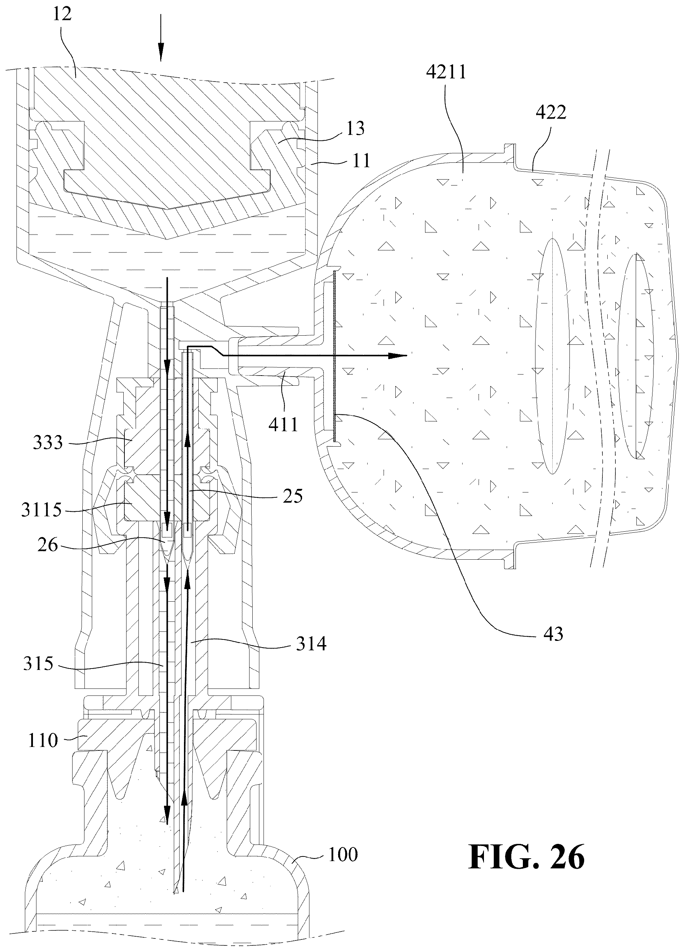

The drug-adding procedure of the sixth embodiment includes the following steps: As shown in FIG. 25 and referring to FIG. 26, the fastener 313 fastens a drug bottle 100, and the pointed tip 3121 of the puncture portion 312 punctures a bottle stopper 110 of the drug bottle 100. At this time, the second connector 30B moves upward, and the first needle 25 sequentially passes through the second stop block 333 and the first stop block 3115 and enters the first flow channel 314. The second needle 26 sequentially passes through the second stop block 333 and the first stop block 3115 and enters the second flow channel 315. In some embodiments, the fastener 313 fastens an infusion end of a soft bag (not shown), and the pointed tip 3121 of the puncture portion 312 punctures the thin film (not shown) at the infusion end of the soft bag. As shown in FIG. 26, the sixth embodiment is on top and the drug bottle 100 or the soft bag at the bottom. The plunger handle 121 is pushed. The plunger 12 drives the piston 13 to move inward, so that the hazardous drug inside the syringe 11 is injected into the drug bottle 100 or the soft bag from top to bottom through the second needle 26 and the second flow channel 315. At the same time, the toxic gas inside the drug bottle 100 or the soft bag passes from bottom to top sequentially through the first flow channel 314, the first needle 25, and the inside of the tube body 411, and passes smoothly through the hydrophobic and breathable membrane 43, and is then injected into the first chamber 4211, while the air bag 422 continues to inflate. Therefore, during the drug-adding procedure, the sixth embodiment can collect toxic gases through the first chamber 4211, maintaining a pressure balance between the internal atmospheric pressure of the drug bottle 100 or soft bag and the external atmospheric pressure. This also prevents small amounts of hazardous drug inside the drug bottle 100 or soft bag from passing through the needle connector 33 in gaseous form or by evaporation. No hazardous drug residue stays on the outer surface of the needle connector 33, and toxic gases will not diffuse into the external space through the needle connector 33. Medical personnel will not come into contact with hazardous drugs on their skin, nor will they inhale toxic gases into their lungs.

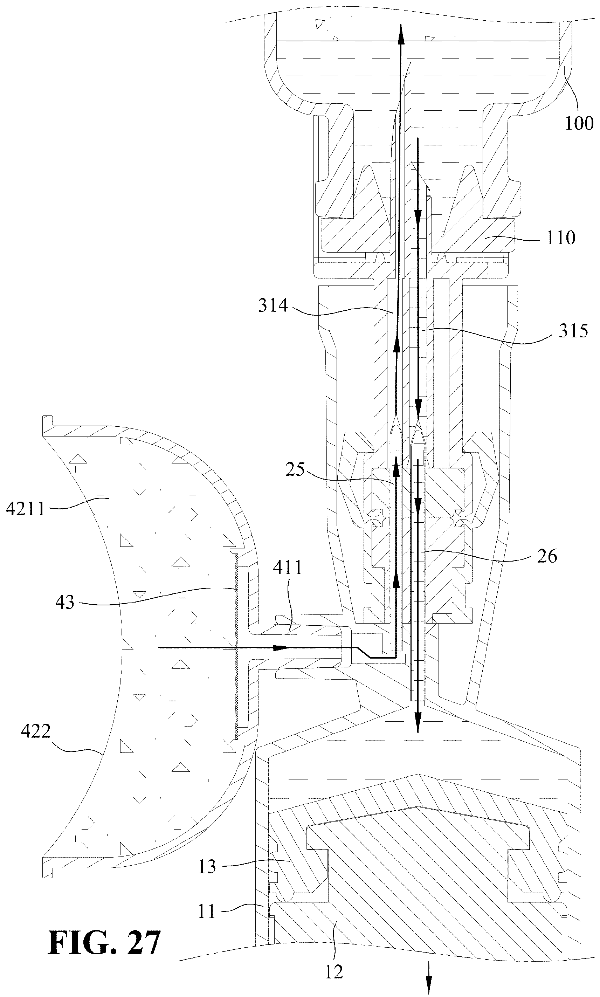

The drug-dispensing procedure of the sixth embodiment includes the following steps: Pushing the plunger handle 121, the plunger 12 drives the piston 13 to move inward, the piston 13 will inject clean air from the inside of the syringe 11 into the first chamber 4211 sequentially through the first flow channel 314, the first needle 25, and the inside of the tube body 411, and the air bag 422 will continue to inflate; as shown in FIG. 25, and referring to FIG. 27, the fastener 313 fastens a drug bottle 100, the pointed tip 3121 of the puncture portion 312 punctures a stopper 110 of the drug bottle 100, at this time the second connector 30B moves upward, the first needle 25 passes sequentially through the second stop block 333 and the first stop block 3115 and enters the first flow channel 314, the second needle 26 passes sequentially through the second stop block 333 and the first stop block 3115 and enters the second flow channel 315; in some embodiments, the fastener 313 fastens an infusion end (not shown) of a soft bag, and the pointed tip 3121 of the puncture portion 312 punctures a thin film (not shown) at the infusion end of the soft bag; as shown in FIG. 27, with the drug bottle 100 or soft bag on top and the sixth embodiment at the bottom, the plunger handle 121 is pulled, and the plunger 12 drives the piston 13 to move outward, so that the hazardous drug inside the drug bottle 100 or soft bag is injected into the syringe 11 from top to bottom through the second flow channel 315 and the second needle 26 in sequence. At the same time, the clean air inside the first chamber 4211 passes smoothly through the hydrophobic and breathable membrane 43 and is injected into the drug bottle 100 or soft bag from bottom to top through the inside of the tube body 411, the first needle 25, and the first flow channel 314 in sequence, and the air bag 422 will continue to contract. Therefore, during the drug-dispensing procedure, the sixth embodiment can collect and supply clean air through the first chamber 4211, maintaining a pressure balance between the internal atmospheric pressure of the drug bottle 100 or soft bag and the external atmospheric pressure, and making it easier to remove hazardous drugs from the drug bottle 100 or soft bag.

Compared to the aforementioned embodiments, the sixth embodiment replaces the first elastic valve 23, conduit 212, and positioning portion 213 of the aforementioned embodiments with a first needle 25 and a second needle 26. Furthermore, the structure of the sleeve 211D in the sixth embodiment is simpler than that of the sleeves 211, 211A, 211B, and 211C in the aforementioned embodiments. Therefore, the overall structure of the sixth embodiment is simpler than that of the aforementioned embodiments, and the manufacturing cost of the sixth embodiment is lower than that of the aforementioned embodiments.

Compared to the previous embodiments, the base 31B of the second connector 30B in the sixth embodiment does not include any components of the pressure balancing devices 40, 40A, and 40B. Therefore, the structure of the base 31B of the second connector 30B in the sixth embodiment is simpler than the structure of the bases 31 and 31A of the second connectors 30 and 30A in the previous embodiments. The manufacturing cost of the base 31B of the second connector 30B in the sixth embodiment is lower than the manufacturing cost of the bases 31 and 31A of the second connectors 30 and 30A in the previous embodiments. When medical personnel are preparing drug, the bases 31B of a plurality of second connectors 30B can be installed on the stoppers of a plurality of drug bottles 100. Medical personnel can then use a set of syringes 10, first connectors 20C, and needle connectors 33 of the second connectors 30B according to the sixth embodiment to connect to the drug bottles 100 through the bases 31B of the second connectors 30B. This eliminates the need to use a plurality of sets of syringes 10, first connectors 20C, and needle connectors 33 of the sixth embodiment, reducing the purchase cost for medical institutions.

Furthermore, because the first flow channel 314 surrounds the outside of the second flow channel 315, the first needle 25 can enter the first flow channel 314 and the second needle 26 can enter the second flow channel 315 regardless of the angle at which the needle connector 33 connects to the base 31B. Directionality is not a concern, preventing errors in direction from affecting operational functionality and improving ease of use and reliability.

Furthermore, during the drug-adding or drug-dispensing procedures and separation operation, the hook portion 2143A can block the second snap-fit portion 3311 to prevent the needle connector 33 from disengaging from the first connector 20C.

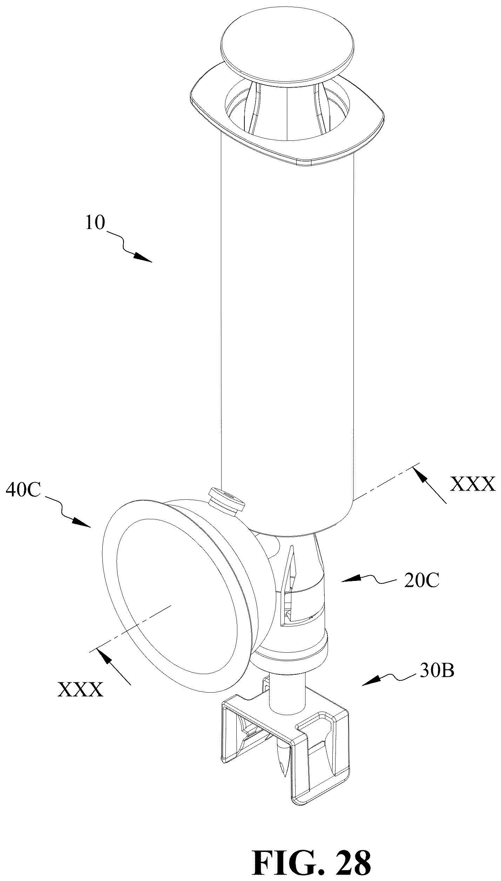

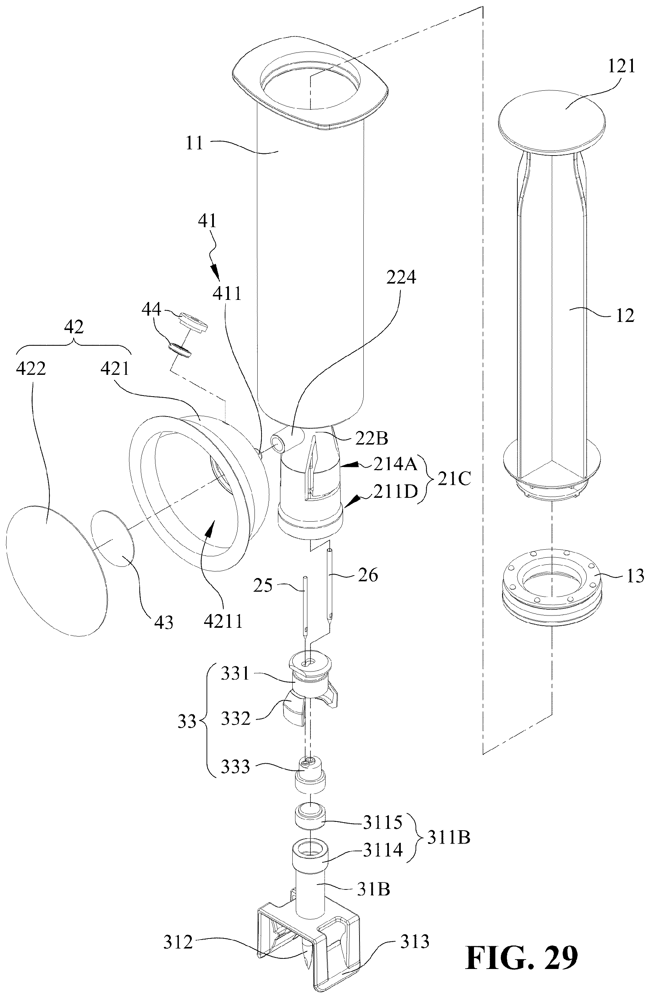

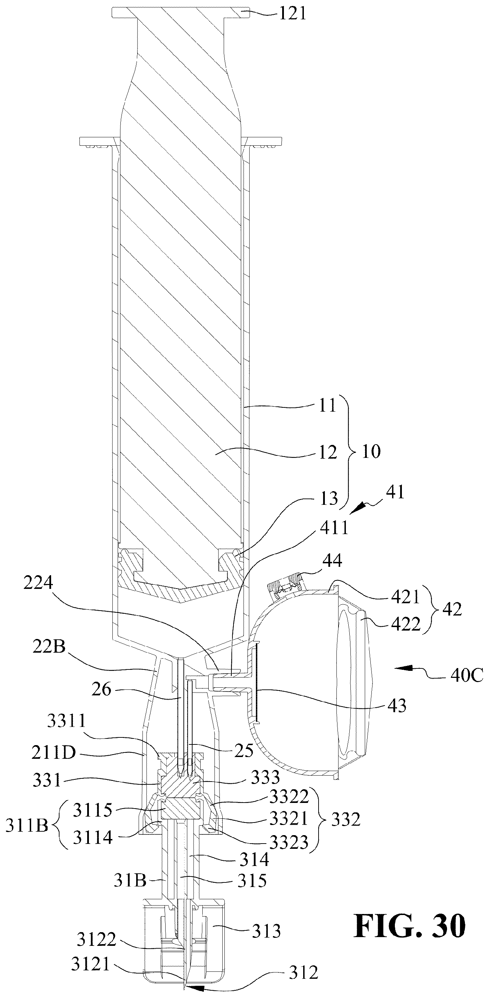

As shown in FIGS. 28, 29, and 30, the difference between the seventh embodiment and the sixth embodiment is that the pressure balancing device 40C includes a check valve 44, which is disposed on the outer cover 421 to allow clean air from the external space to enter the first chamber 4211 and to prevent air from flowing out of the first chamber 4211.

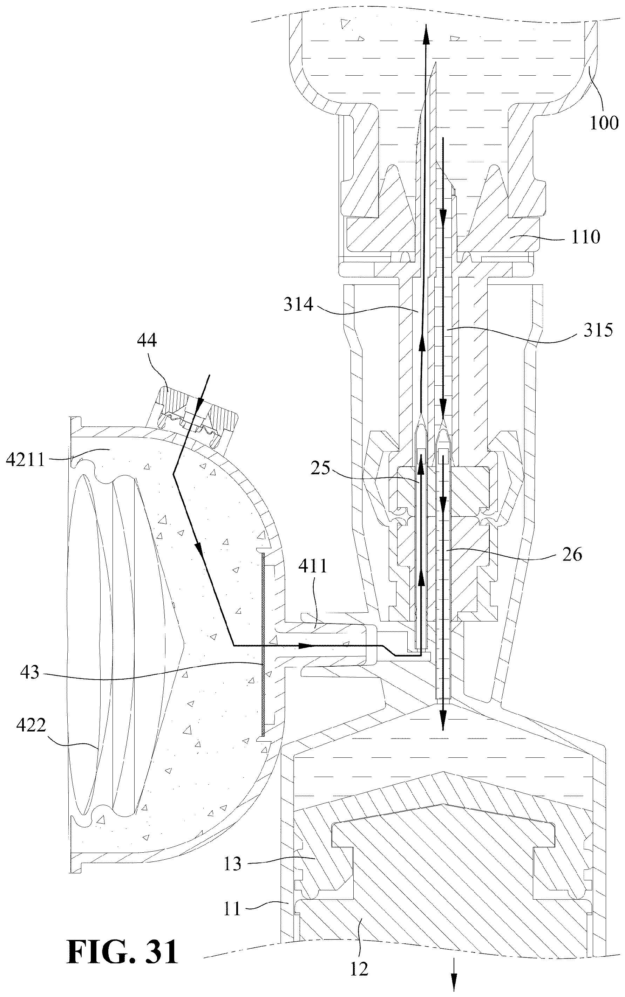

As shown in FIG. 31, the difference between the drug-dispensing procedure of the seventh embodiment and that of the sixth embodiment is that after the airbag 422 contracts, the clean air in the first chamber 4211 is exhausted, and clean air from the external space enters the first chamber 4211 through the check valve 44. The clean air inside the first chamber 4211 passes smoothly through the hydrophobic and breathable membrane 43 and then sequentially passes from bottom to top through the inside of the tube body 411, the first needle 25, and the first flow channel 314 into the inside of the drug bottle 100 or soft bag. Therefore, during the drug-dispensing procedure, the seventh embodiment can collect and provide clean air through the first chamber 4211. After the clean air in the first chamber 4211 is exhausted, clean air is supplied by the check valve 44, so that the atmospheric pressure inside the drug bottle 100 or soft bag can maintain a pressure balance with the external atmospheric pressure, and the hazardous drugs can be easily removed from the inside of the drug bottle 100 or soft bag. Furthermore, if moisture from the external space enters the first chamber 4211 through the check valve 44, the moisture will be blocked by the hydrophobic and breathable membrane 43 and thus avoiding moisture entering the drug bottle 100 or the soft bag and mixing with the drug, causing contamination or dilution.

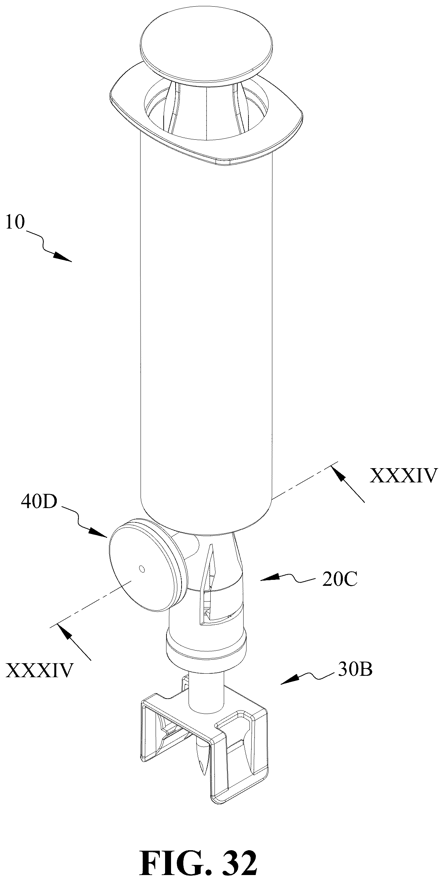

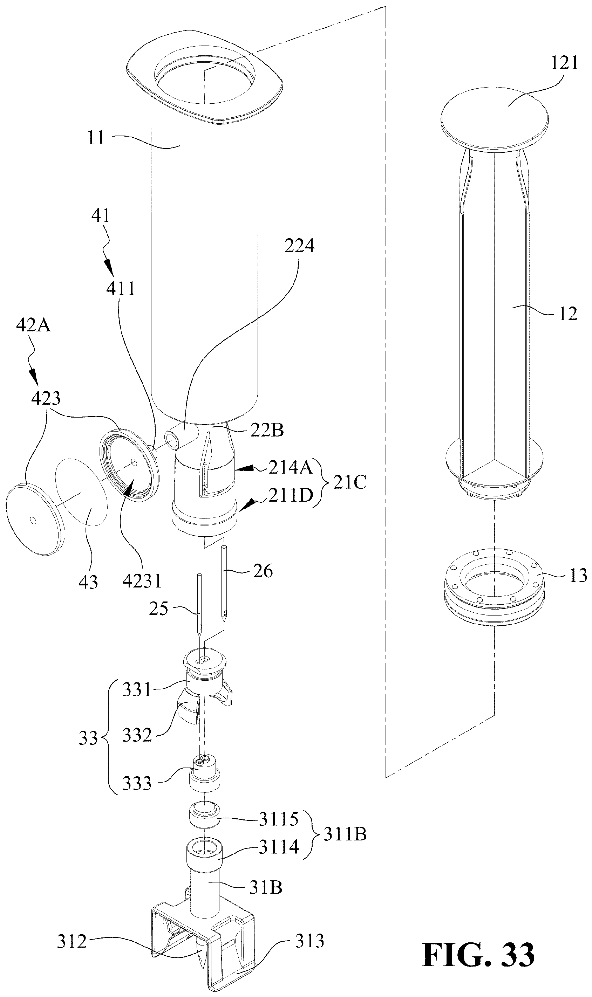

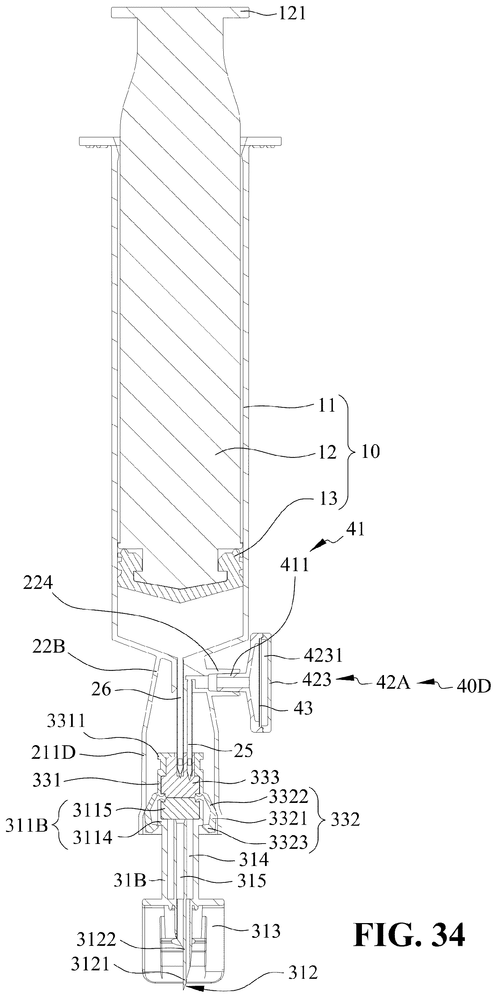

As shown in FIGS. 32, 33, and 34, the difference between the eighth embodiment and the sixth embodiment is that the collecting portion 42A of the pressure balancing device 40D includes a filter 423. The filter 423 is integrally formed with one end of the tube body 411 and includes a second chamber 4231. The inside of the tube body 411 communicates with the second chamber 4231. The hydrophobic and breathable membrane 43 is disposed in the second chamber 4231 and near the opening of the tube body 411. Specifically, some drugs are non-toxic and do not produce toxic gases. When these drugs are used for drug preparation, medical personnel can choose the method described in the eighth embodiment. In this case, the filter 423 has the following two advantages: First, the filter 423 seals the first flow channel 314, preventing it from communicating with the external space and preventing air and moisture in the external space from entering the drug bottle 100 or soft bag and mixing with the drug. Second, the filter 423 provides a smaller second chamber 4231, which can collect a small amount of clean gas from the drug bottle 100 or soft bag, maintaining a pressure balance between the internal atmospheric pressure and the external atmospheric pressure. Third, the manufacturing cost of the filter 423 is lower than that of the outer cover 421 and the airbag 422.

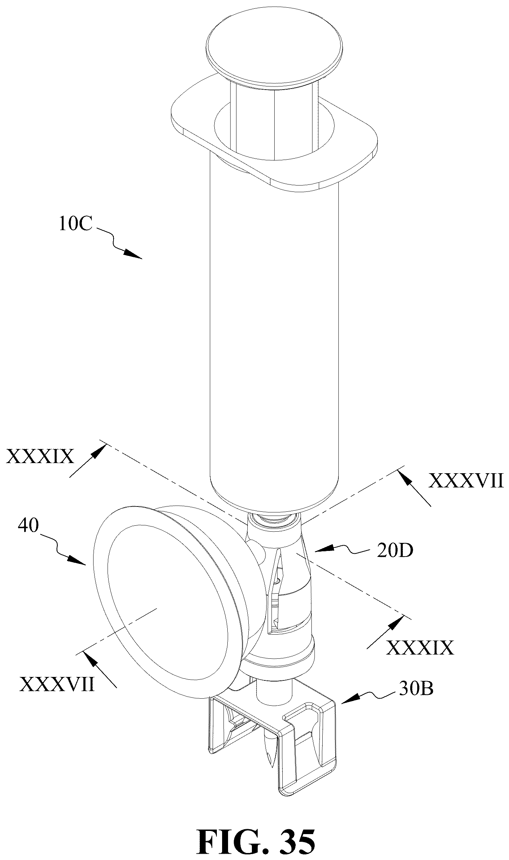

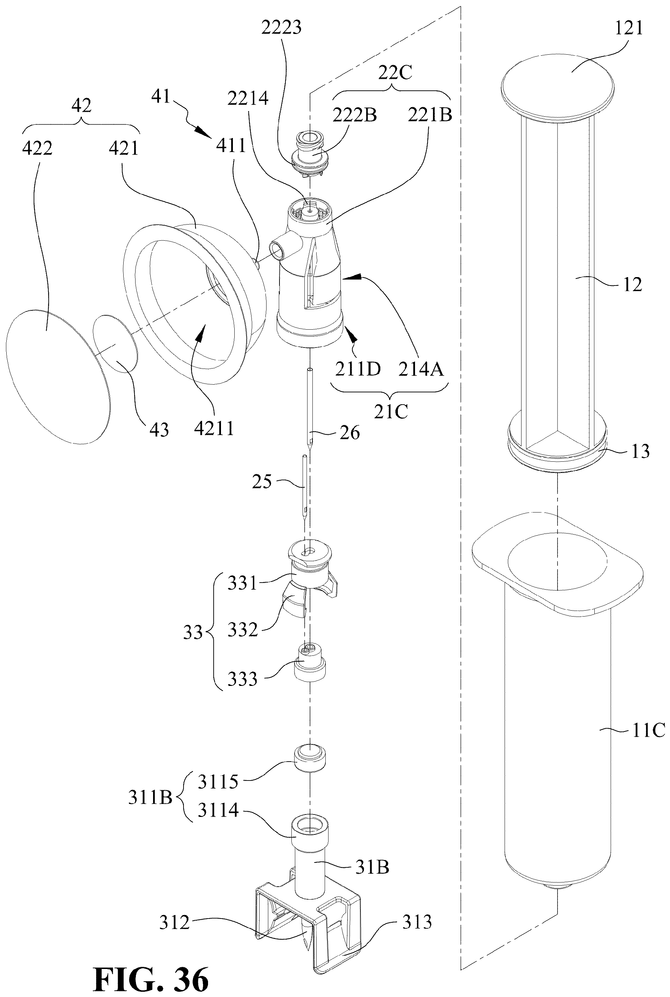

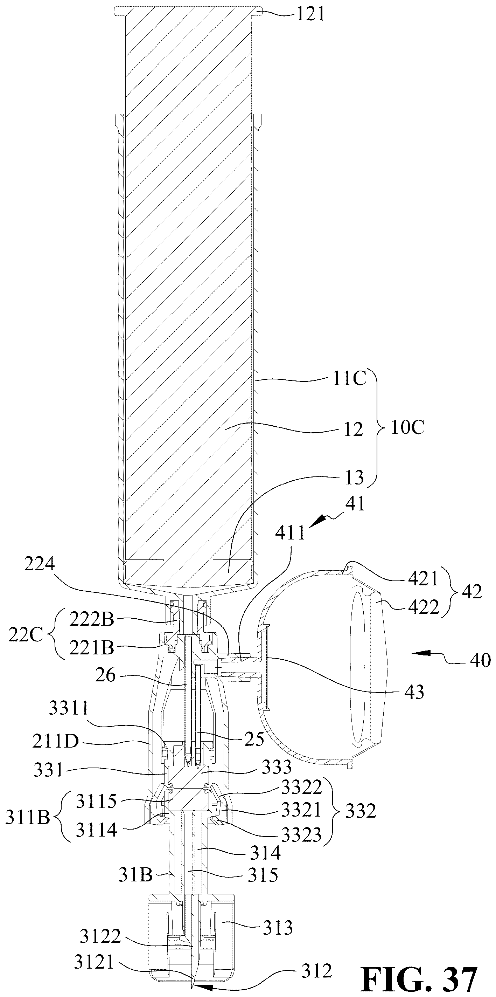

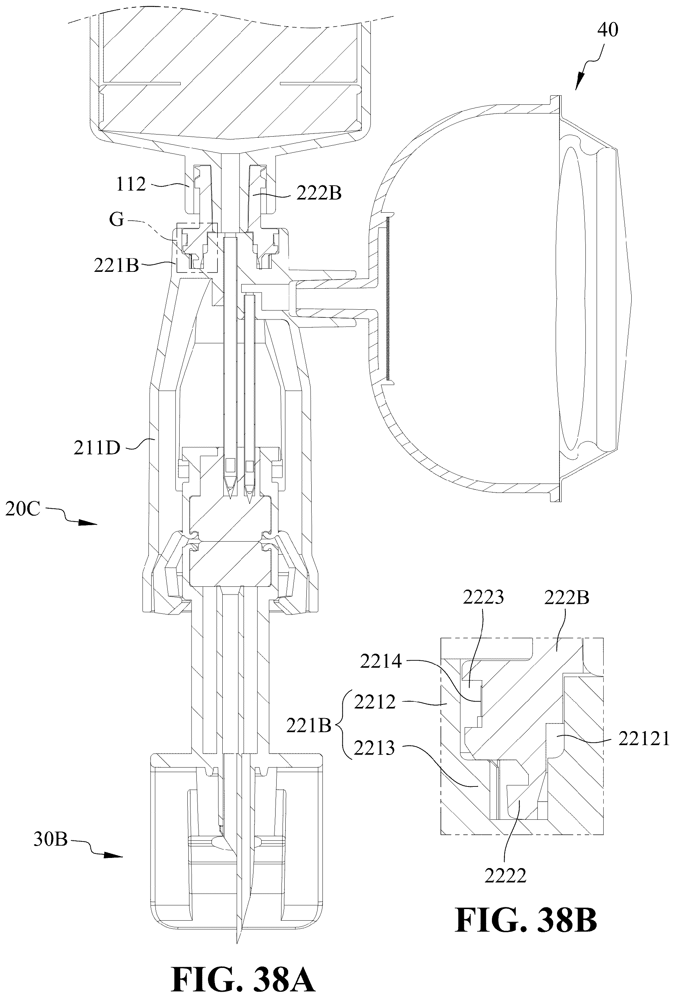

As shown in FIGS. 35 to 39B, the differences between the ninth embodiment and the sixth embodiment are as follows: First, the first engaging portion 22C of the first connector 20D includes a first end 221B and a second end 222B. The first end 221B is integrally formed with the top end of the sleeve 211D. The first snap-fit portions 214A connect the first end 221B, and the first needle 25 and the second needle 26 are inserted into the first end 221B. Second, the first end 221B has an outer peripheral wall 2212, a plurality of teeth 2213, and a plurality of sliders 2214. The outer peripheral wall 2212 forms a receiving groove 22121. The teeth 2213 are arranged around the inner side of the outer peripheral wall 2212, and the sliders 2214 are arranged around the inner side of the outer peripheral wall 2212 and located above the teeth 2213. Third, the bottom of the second end 222B is received in the receiving groove 22121, the second end 222B has a plurality of pawls 2222 and a ring groove 2223, the pawls 2222 are arranged around the outer side of the bottom of the second end 222B and engage with the teeth 2213, the ring groove 2223 is arranged around the outer side of the second end 222B and located above the pawls 2222, the sliders 2214 are located in the ring groove 2223, the top of the second end 222B has an external thread, and a connector 112 at the bottom end of the syringe 11C of the syringe 10C has an internal thread.

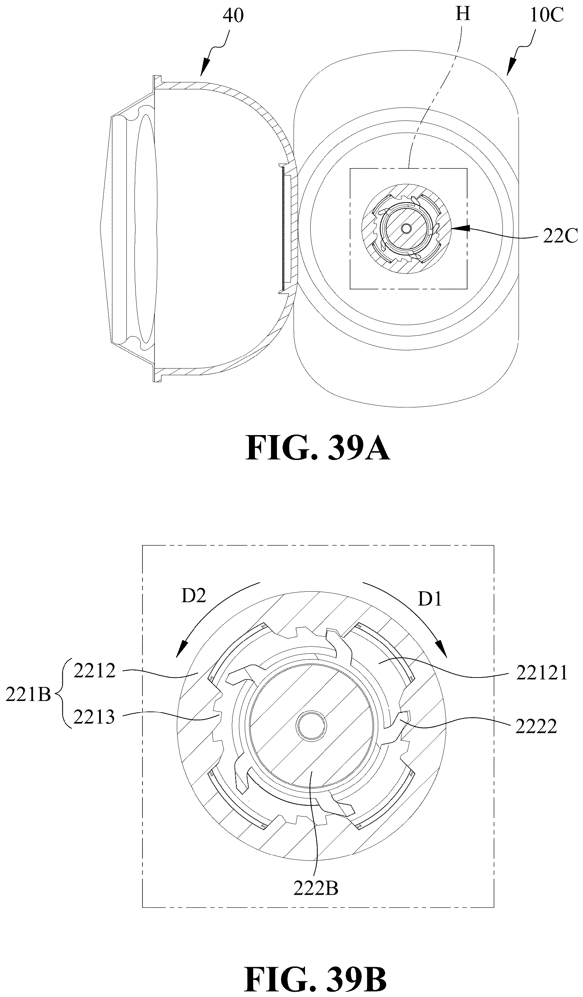

As shown in FIGS. 39A and 39B, when the first end 221B rotates along a first direction D1 by a first rotational force, the first end 221B sequentially transmits the first rotational force to the second end 222B through the teeth 2213 and the pawls 2222. The second end 222B is pushed by the first rotational force and rotates along the first direction D1, so that the top of the second end 222B is fixed to the bottom end of the syringe 11C.

As shown in FIGS. 39A and 39B, after the top of the second end 222B is fixed to the bottom of the syringe 11C, when the first end 221B rotates along a second direction D2 opposite to the first direction D1 by a second rotational force, the first end 221B uses the second rotational force to press the pawls 2222 through the teeth 2213, causing the pawls 2222 to bend and deform to disengage from the teeth 2213, and the first end 221B rotates freely relative to the second end 222B along the second direction D2.

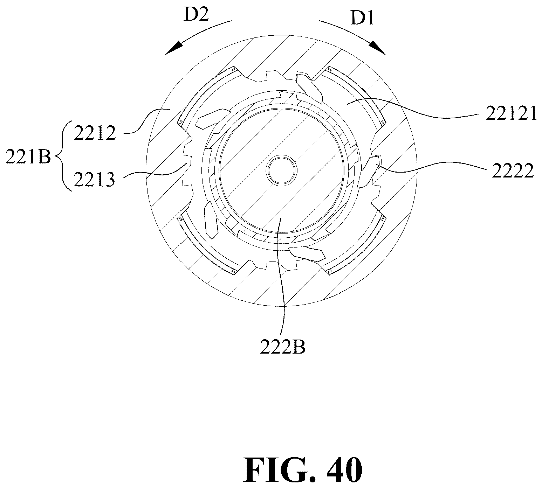

As shown in FIG. 40, after the top of the second end 222B is fixed to the bottom of the syringe 11C, when the first end 221B rotates along the first direction D1 by a third rotational force, the first end 221B transmits the third rotational force to the pawls 2222 through the teeth 2213. The third rotational force is greater than the force that the pawls 2222 can withstand, and the pawls 2222 are broken, so that the second end 222B can rotate arbitrarily relative to the first end 221B along the first direction D1 and the second direction D2.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A hazardous drug liquid-gas exchange device, comprising:
a syringe, comprising a syringe barrel, a plunger, and a piston, the plunger being disposed within the syringe barrel and having a plunger handle at a top end, the piston being disposed at a bottom end of the plunger and located within the syringe barrel;
a first connector, disposed at the bottom end of the syringe barrel;
a second connector, capable of being connected to or separate from the first connector and having a puncture portion, and being disposed with a first flow channel and a second flow channel, the puncture portion having a pointed tip at a bottom end, and the first and second flow channels penetrating the bottom end of the puncture portion; and
a pressure balancing device, comprising a guide portion, a collecting portion, and a hydrophobic and breathable membrane; the guide portion being disposed on the first connector and/or the second connector and communicating with the first flow channel, the collecting portion communicating with the guide portion, and the hydrophobic and breathable membrane being disposed on the guide portion and/or the collecting portion.

2. The hazardous drug liquid-gas exchange device according to claim 1, wherein the first connector further comprises a body, a first engaging portion, a first needle, and a second needle; the body comprises a sleeve and two first snap-fit portions, the sleeve has two first through holes, the first snap-fit portions are respectively located in the first through holes, the first engaging portion is disposed at the top of the sleeve and the bottom of the syringe, the first snap-fit portions connect to the first engaging portion, the first needle and the second needle are inserted into the first engaging portion, and the second needle communicates with the inside of the syringe;
wherein the second connector comprises a base and a needle connector, the base comprises an assembly portion and a puncture portion and is disposed with a first flow channel and a second flow channel; the assembly portion comprises a mounting base and a first stop block, the first stop is disposed inside the mounting base and seals the openings of the first and second flow channels; the needle connector comprises a housing, two second engaging portions, and a second stop block, the housing has a second snap-fit portion located on the outer side of the housing, the second snap-fit portions are located at the bottom end of the housing, the second stop block is located inside the housing, the mounting base is fixed to the second engaging portions;
wherein the guide portion is located on the first engaging portion, and the first needle communicates with the guide portion;
wherein when the first connector mates with the needle connector, the first snap-fit portions are fixed to the second snap-fit portions, and the first and second needles are inserted into the second stop block but do not penetrate the second stop block; and
wherein when the second connector moves upward, the first needle sequentially penetrates the second stop block and the first stop block and enters the first flow channel, and the second needle sequentially penetrates the second stop block and the first stop block and enters the second flow channel.

3. The hazardous drug liquid-gas exchange device according to claim 2, wherein the first engaging portion has a protruding portion, the guide portion comprises a tube body connected to the protruding portion of the first engaging portion, the inside of the conduit communicates with the inside of the first needle, the collecting portion comprises an outer cover and an air bag, the outer cover is integrally formed with one end of the tube body, the outer cover has a first chamber, the inside of the tube body communicates with the first chamber, a hydrophobic and breathable membrane is disposed in the first chamber and near the opening of the tube body, and the air bag is disposed at the opening of the first chamber.

4. The hazardous drug liquid-gas exchange device according to claim 3, wherein the pressure balancing device comprises a check valve disposed on the outer cover for allowing clean air from the external space to enter the first chamber and for preventing air from flowing out of the first chamber.

5. The hazardous drug liquid-gas exchange device according to claim 2, wherein the first flow channel surrounds the outside of the second flow channel.

6. The hazardous drug liquid-gas exchange device according to claim 2, wherein the guide portion comprises a tube body disposed on one side of the first engaging portion, the inside of the tube body communicates with the inside of the first needle; the collecting portion comprises a filter integrally formed with one end of the tube body and is disposed with a second chamber, the inside of the tube body communicates with the second chamber; and a hydrophobic and breathable membrane is disposed in the second chamber and near the opening of the tube body.

7. The hazardous drug liquid-gas exchange device according to claim 2, wherein the first engaging portion is integrally formed with the top end of the sleeve and the bottom end of the syringe.

8. The hazardous drug liquid-gas exchange device according to claim 2, wherein the first engaging portion comprises a first end and a second end; the first end is integrally formed with the top end of the sleeve, the first snap-fit portions connect to the first end, the first needle and the second needle are inserted into the first end; the first end has an outer peripheral wall, a plurality of teeth, and a plurality of sliders, the outer peripheral wall forms a receiving groove, the teeth are arranged around the inner side of the outer peripheral wall, and the sliders are arranged around the inner side of the outer peripheral wall and located above the teeth; the bottom of the second end is received in the receiving groove, the second end has a plurality of pawls and a ring groove, the pawls are arranged around the outside of the bottom of the second end and engage with the teeth, the ring groove is arranged around the outside of the bottom of the second end and is located above the pawls, and the sliders are located in the ring groove; the top of the second end has an external thread, and a connector at the bottom end of the syringe has an internal thread;
wherein when the first end rotates along a first direction by a first rotational force, the second end sequentially transmits the first rotational force to a second end via the teeth and pawls, the second end is pushed by the first rotational force and rotates along a first direction, such that the top of the second end is fixed to the bottom of the syringe;
wherein after the top of the second end is fixed to the bottom of the syringe, when the first end rotates along a second direction opposite to the first direction by a second rotational force, the first end uses the teeth to press the pawls with the second rotational force, causing the pawls to bend and deform, disengaging from the teeth; the first end then rotates freely relative to the second end along the second direction;
wherein after the top of the second end is fixed to the bottom of the syringe, when the first end rotates along the first direction by a third rotational force, the first end transmits the third rotational force to the pawls via the teeth, the third rotational force exceeds the force that the pawls can withstand, causing the pawls to break, allowing the second end to rotate arbitrarily relative to the first end along both the first and second directions.

9. The hazardous drug liquid-gas exchange device according to claim 1, wherein the first connector is a movable closed connector, comprising a body, a first engaging portion, and a first elastic valve; the body comprises a sleeve, a conduit, a positioning portion, and two first snap-fit portions; the conduit is disposed inside the sleeve portion and has a first channel and a first opening, the positioning portion is located inside the sleeve portion and disposed outer side the conduit, the first snap-fit portions are disposed at the outside the sleeve portion, the first engaging portion is disposed at the top of the sleeve portion and the bottom of the syringe, the first elastic valve comprises a first circumferential portion and a first sealing portion, the two ends of the first circumferential portion are respectively disposed at the positioning portion and the first engaging portion, the first sealing portion is located in the first channel, the top end of the first sealing portion is connected to the inner side of the first circumferential portion and is disposed with a plurality of perforations, the perforations communicate with the first channel, and the bottom end of the first sealing portion closes the first opening; wherein the second connector is a snap-fit, closed, needle-free connector module and comprises a base and a needle-free connector; the base comprises an assembly portion and a piercing portion and is disposed with a first flow channel and a second flow channel; the assembly portion has an assembly groove and a shaft hole, the second flow channel communicates with the shaft hole; the needle-free connector comprises a housing, a second engaging portion, and a second elastic valve, the housing is disposed with a second snap-fit portion at the outside, the top end of the housing has a second opening, the second engaging portion is located on the outer side of the housing and attached to the outside of the assembly portion, the second elastic valve comprises a second circumferential portion and a second sealing portion, the second circumferential portion is disposed in the assembly groove and has a second channel communicating with the shaft hole, the second sealing portion is disposed inside the housing and has a slit, and the second sealing portion closes the second opening; wherein the guide portion is disposed on the body and/or the base; wherein when the first connector mates with the needle-free connector, the first snap-fit portion is fixed to the second snap-fit portion, the guide portion compresses the second sealing portion, and the second sealing portion compresses the second circumferential portion, causing the second sealing portion to move away from the second opening, and the guide portion passes through the slit of the second sealing portion and enters the second sealing portion, the top of the housing abuts against the inner wall of the sleeve, the positioning portion moves along the conduit away from the needle-free connector and compresses the first circumferential portion, causing the bottom end of the first sealing portion to disengage from the first opening, and the first channel communicates with the second channel; and when the first connector separates from the needle-free connector, the first snap-fit portions disengage from the second snap-fit portions, the conduit disengages from the second sealing portion, causing the slit of the second sealing portion to close, and the second sealing portion resets and closes the second opening due to the elasticity of the second circumferential portion; the top of the housing disengages from the sleeve, and the positioning portion and the first sealing portion reset due to the elasticity of the first circumferential portion, causing the bottom end of the first sealing portion to close the first opening.

10. The hazardous drug liquid-gas exchange device according to claim 9, wherein the base is disposed with a protruding portion, the guide portion comprises a tube body connected to the protruding portion of the base, the inside of the tube body communicates with the first flow channel, the collecting portion comprises an outer cover and an air bag, the outer cover being integrally formed with one end of the tube body, the outer cover is disposed with a first chamber, the inside of the tube communicates with the first chamber, a hydrophobic and breathable membrane is disposed in the first chamber and near the opening of the tube body, and the air bag is disposed at the opening of the first chamber.

11. The hazardous drug liquid-gas exchange device according to claim 9, wherein the syringe further comprises a cover disposed at the top of the syringe and having an air passage communicating with the inside of the syringe, such that the air passage and the inside of the syringe together form the collecting portion, the plunger passes through the cover, and the plunger handle is located above the cover; wherein the inner diameter of the first end of the housing is larger than the outer diameter of the conduit; wherein the guide portion comprises a tube body and a filter, the top end of the tube is disposed on one side of the cover, and the bottom end of the tube body is disposed on one side of the sleeve, and the inside of the tube body communicates with the air passage and the inside of the sleeve; the filter is disposed on one side of the base and is disposed with a second chamber, an inlet, and an outlet; the second chamber communicates with the inlet and the outlet, and the inlet communicates with the first flow channel; the assembly portion is disposed with a third flow channel, and the outlet communicates with the third flow channel; a fourth flow channel is formed on the inner wall of the assembly groove, and the fourth flow channel communicates with the third flow channel, and the hydrophobic and breathable membrane is disposed in the second chamber, wherein the hazardous drug liquid-gas exchange device further comprises a sealing ring, disposed inside the sleeve and abutting against the top outer side of the housing, or disposed on the top outer side of the housing and abutting against the inside of the sleeve; wherein when the first connector mates with the needle-free connector, the inside of the sleeve communicates with the second opening, the second opening communicates with the inside of the housing, and the inside of the housing communicates with the fourth flow channel.

12. The hazardous drug liquid-gas exchange device according to claim 11, wherein the cover is disposed a protruding portion on one side, the protruding portion of the cover opening the air passage; the syringe is disposed with a groove on one side of top end, and the protruding portion of the cover is fixed in the groove; the top end of the tube body is connected to the protruding portion of the cover, the sleeve is disposed with a protruding portion on one side, and the bottom end of the tube body is connected to the protruding portion of the sleeve.

13. The hazardous drug liquid-gas exchange device according to claim 11, wherein the base is disposed with a first insertion portion and a second insertion portion on one side, the filter is disposed with an inlet end and an outlet end, the inlet end is inserted into the first insertion portion and forms the inlet, and the outlet end is inserted into the second insertion portion and forms the outlet.

14. The hazardous drug liquid-gas exchange device according to claim 9, wherein the pressure balancing device comprises a first hydrophobic and breathable membrane, a second hydrophobic and breathable membrane, and a check valve; the check valve is located on one side of the sleeve to allow clean air from the external space to enter the sleeve and to prevent air from flowing out of the sleeve; the collecting portion comprises an outer cover and an air bag, the outer cover is disposed with a first chamber, and the airbag is located at the opening of the first chamber; the guide portion comprises a tube body and a filter, the tube body is located on one side of the sleeve, and the inside of the tube body communicates with the inside of the sleeve; the outer cover is integrally formed with one end of the tube body, and the inside of the tube body communicates with the first chamber; the first hydrophobic and breathable membrane is located in the first chamber and near the opening of the tube body, the filter is disposed on one side of the base and is disposed with a second chamber, an inlet, and an outlet, the second chamber communicates with the inlet and the outlet, and the inlet communicates with the first flow channel; the assembly portion is disposed with a third flow channel, and the outlet communicates with the third flow channel; a fourth flow channel is formed on the inner wall of the assembly groove, and the fourth flow channel communicates with the third flow channel; the second hydrophobic and breathable membrane is disposed in the second chamber; wherein the first connector further comprises a sealing ring disposed inside the sleeve and outside the housing; wherein when the first connector mates with the needle-free connector, the inside of the sleeve communicates with the second opening, the second opening communicates with the inside of the housing, and the inside of the housing communicates with the fourth flow channel.

15. The hazardous drug liquid-gas exchange device according to claim 14, wherein the sleeve has a protruding portion on one side, and the tube body is connected to the protruding portion of the sleeve.

16. The hazardous drug liquid-gas exchange device according to claim 14, wherein one side of the base is disposed with a first insertion portion and a second insertion portion, the filter is disposed with an inlet end and an outlet end, the inlet end is inserted into the first insertion portion and forms the inlet, and the outlet end is inserted into the second insertion portion and forms the outlet.

17. The hazardous drug liquid-gas exchange device according to claim 9, wherein the outer side of the first engaging portion is connected to the inner side of the sleeve.

18. The hazardous drug liquid-gas exchange device according to claim 9, wherein the outer side of the assembly portion is connected to the inner side of the housing.

19. The hazardous drug liquid-gas exchange device according to claim 9, further comprising; a filter air guide cover, which further comprising a base, an outer circumferential wall, an inner circumferential wall, and a filter paper; the base is disposed with a receiving groove, the outer circumferential wall and the inner circumferential wall are disposed on the base, the inner circumferential wall is located inside the outer circumferential wall, the height of the outer circumferential wall is greater than the height of the inner circumferential wall, the inner circumferential wall is disposed with an air guide hole; a pushing portion is disposed protrudingly from the top of the inner circumferential wall; the air guide hole passes through the pushing portion and the base and communicates with the receiving groove; the filter paper is disposed in the receiving groove; wherein when the first connector is connected to the filter air guide cover, the base and the outer circumferential wall abut against the sleeve, the pushing portion pushes the first sealing portion to bend and deform, so that the bottom end of the first sealing portion is separated from the first opening, and the first channel communicates with the air guide hole; wherein when the first connector is separated from the filter air guide cover, the first sealing portion returns to original shape and closes the first opening.
